# EUROPEAN PATENT SPECIFICATION

(11) **EP 3 232 944 B1**
(45) Date of publication and mention of the grant of the patent: **01.07.2020**
(21) Application number: 15870789.3
(22) Date of filing: 14.12.2015
(51) Int. Cl.: A61B 17/04, A61B 17/00

(54) **MULTIPLE LOOP SURGICAL SNARE ASSEMBLY**
CHIRURGISCHE SCHLINGENANORDNUNG MIT MEHREREN SCHLEIFEN
ENSEMBLE ANSE CHIRURGICALE À BOUCLES MULTIPLES

(30) Priority: 15.12.2014 US 201462091748 P; 26.05.2015 US 201514721048
(43) Date of publication of application: 25.10.2017
(73) Proprietor: LSI Solutions, Inc., Victor, NY 14564 (US)
(72) Inventor: SAUER, Jude, S., Pittsford, NY 14534 (US)
(74) Representative: Flügel Preissner Schober Seidel
(86) International application number: PCT/US2015/065510
(87) International publication number: WO 2016/100191

(56) References cited:
- US-A- 5 562 685
- US-A1- 2004 034 369
- US-A1- 2006 161 183
- US-A1- 2006 271 060
- US-A1- 2012 283 749
- US-A1- 2012 283 749
- US-A1- 2014 276 979
- US-A1- 2014 276 979
- US-B1- 6 171 317
- US-B1- 6 171 317

## Description

### FIELD

This invention relates generally to surgical suturing and more particularly to a surgical snare assembly having proximal and distal suture engaging loops.

### BACKGROUND

Minimally invasive surgery (MIS) has allowed physicians to carry out many surgical procedures with less resultant patient pain and disability than conventional, open surgery. However, unlike conventional open surgery, where the surgical site is readily accessible through a large incision, MIS typically requires the surgeon to operate remotely by inserting and manipulating instruments through small punctures, openings, or access sites in the body wall. For example, a hollow cannula may be placed in a small puncture to create a minimally invasive entry point. Cannulas, typically have tubular passages with a diameter ranging in size from 5 to 20 millimeters (mm) and are often sealed to maintain positive pressure within the peritoneal cavity to facilitate pneumoperitoneum during laparoscopic surgery. One or more cannulas may be inserted into the body for any given operation. Medical instruments, such as grippers, cutters, suturing (sewing) devices, etc., are then inserted through the one or more cannulas and manipulated directly by a surgeon's hands or indirectly by robotic interface.

MIS relies on very specialized surgical skills, knowledge, tools, and teamwork. One particularly challenging aspect of MIS procedures involves suture placement. Alternative techniques for MIS suture placement, especially those employing mechanically assisted suturing devices, often provide two suture ends left untied on one side of a wound closure site or at a prosthetic attachment site. Accordingly, various MIS suture fastening devices are available to restrain the untied suture ends. For example, the COR-KNOT® and Ti-KNOT® devices, available from LSI Solutions, Inc. of Victor, NY, can apply mechanical knots to the untied suture ends in order to help complete the wound closure or prosthetic attachment.

Since mechanical knots and the like are often quite small, single loop snares have been developed to pull the untied suture ends through a mechanical knot before the knot is fastened to hold the suture in place. This works very well when a surgeon is manipulating the mechanical knotting device and is available to control the suture tension outside of a patient's body while the knotting device is subsequently inserted into the body for knot application. Unfortunately, in many modern procedures, surgeons often do not have hands available outside the patient's body since their hands may otherwise be occupied by robotic surgical controls or other instruments which are active inside the patient's body during the MIS procedure. Non-surgeon surgical assistants may be available, but are usually not trained or allowed to tension the suture prior to knot placement. Therefore, a second surgeon must often be available to assist with the knotting process, or the primary surgeon must temporarily ignore the internal instruments while knotting is completed. In the first case, the second surgeon's skills and time are diverted from being able to help another patient, and in the second case, the unattended internal instruments may present risk to the patient. Furthermore, such techniques do not enable the running of one or more additional lengths of suture from a separate, anchored closed loop of suture.

US 2012/283749 A1 discloses a snare assembly having a pledget. Two surgical loops are guided through the pledget.

US 5,562,685 A discloses a surgical instrument employed in suturing tissue at a remote location such as in laparoscopic surgery.

US 2014/276979 A1 refers to a device for securing bi-directional suture loops using coaxial mechanical fasteners. The surgical snare assembly shown in US 2014/276979 A1 comprises two loops, wherein one loop is guided in an opposite direction through the suture fastener compared to the other loop. This is done for drawing another suture through the suture fastener.

US 2004/034369 A1 refers to a system for endoscopic suturing.

Therefore, there is a need for a surgical snare assembly and related methods which are compatible with commercially available suture knotting technology while enabling a single surgeon to tension a suture internally for knotting without the need for a second surgeon to apply external tension during an MIS procedure. There is also a need for a surgical snare assembly and related methods which will enable the running of one or more additional lengths of suture from a separate, anchored closed loop of suture in a minimally invasive surgical procedure.

### SUMMARY

A surgical snare assembly is disclosed. The surgical snare assembly has a suture fastener having an entrance and an exit. The surgical snare assembly also has a proximal suture engaging loop and a distal suture engaging loop. The surgical snare assembly further has a handle which is directly coupled to the proximal suture engaging loop and indirectly coupled to the distal suture engaging loop, and configured such that: 1) movement of the handle a first distance away from the suture fastener causes the proximal suture engaging loop to move through the suture fastener from the entrance to the exit; and 2) movement of the handle a second distance away from the suture fastener causes the distal suture engaging loop to move through the suture fastener from the entrance to the exit. The distal suture engaging loop is indirectly coupled to the handle by the proximal suture engaging loop and a twisted filament extending between the proximal suture engaging loop and the distal suture engaging loop, or by the proximal suture engaging loop and at least one or more filaments extending between the proximal suture engaging loop and the distal suture engaging loop

### BRIEF DESCRIPTION OF THE DRAWINGS

FIG. 1 is a distally oriented perspective view of a surgical pledget assembly.
FIG. 2 is a proximally oriented perspective view of the pledget assembly of FIG. 1 as presented for use.
FIG. 3 is a perspective view of the pledget assembly presented in FIG. 1 in preparation for use in a corresponding application instrument when applied to its field of use.
FIG. 4 is a perspective view of the distal end of the pledget assembly and corresponding application instrument presented in FIG. 3 as applied to its intended field of use.
FIG. 5 is a proximal perspective view of the pledget assembly accepting suture tails from a wound site.
FIG. 6 is a proximal perspective view of the pledget assembly of FIG. 5 illustrating the initial passing of suture through the device.
FIG. 7 is a proximal perspective view of the pledget assembly of FIG. 6 illustrating the final stage of progression of the suture through the device.
FIG. 8 is a proximal perspective view of the pledget assembly of FIG. 7 demonstrating the suture completely passed through the device.
FIG. 9 is a proximal perspective view of the pledget assembly of FIG. 8 pressed against the wound site and closing the wound area via the tension of the suture.
FIG. 10 is a perspective view of the closed wound with applied suture securing sleeve.
FIG. 11 is an exploded perspective view detailing a representative sequential step in the assembly of the pledget assembly.
FIG. 12 is a perspective view of the pledget assembly disclosed in FIG. 11.
FIG. 13 is a perspective view illustrating the initialization of the forming process for the pledget assembly of FIG. 12.
FIG. 14 is a perspective view of the formed pledget assembly of FIG. 13.
FIG. 15 is an exploded perspective view of an additionally formed and adapted pledget assembly of FIG. 14.
FIG. 16 is a perspective view illustrating partial assembly of the pledget assembly illustrated in FIG. 15.
FIG. 17 is a perspective view of an example of a pledget assembly illustrating a variation of the installed ends of the wire snare.
FIG. 18 is a perspective view of an example of a pledget assembly wherein two separate wire snare loops are passed individually through a pledget.
FIGS. 19A-19J schematically illustrate different examples and embodiments of a surgical snare assembly in partial cross-sectional view. Embodiments of the invention are illustrated in figures 19G-19H.
FIG. 20 schematically illustrates another example of a surgical snare assembly in partial cross-sectional view.
FIG. 21A illustrates one embodiment of a surgical snare assembly having a proximal holder for the proximal suture engaging loop.
FIG. 21B illustrates one embodiment of a surgical snare assembly having a proximal holder for the proximal suture engaging loop and a distal holder for the distal suture engaging loop.
FIGS. 22A-22G illustrate one embodiment of a surgical snare assembly in use with a suture fastener applicator to enable internal tensioning of a suture stitch while an assistant anchors one end of the suture externally.
FIGS. 23A-23G illustrate one embodiment of a surgical snare assembly in use with a suture fastener applicator to enable internal threading of one end of a second suture through a mechanical fastener being applied to first and second ends of a first suture sewn internally and held externally.
FIGS. 24A-24G illustrate one embodiment of a surgical snare assembly in use with a suture fastener applicator to enable internal tensioning of a first end of a first suture while an assistant externally anchors a second end of the first suture, and while enabling internal threading of one end of a second suture through a mechanical fastener being applied to the first and second ends of the first suture.
FIG. 25 illustrates one embodiment of a surgical kit having a suture fastener applicator and at least one surgical snare assembly.
FIGS. 26A and 26B illustrate embodiments of a suture fastener having and entrance and an exit for use with a surgical snare assembly.
FIGS. 27-30 illustrate examples of suturing methods.

It will be appreciated that for purposes of clarity and where deemed appropriate, reference numerals have been repeated in the figures to indicate corresponding features, and that the various elements in the drawings have not necessarily been drawn to scale in order to better show the features.

### DETAILED DESCRIPTION

### Pledgetted Examples

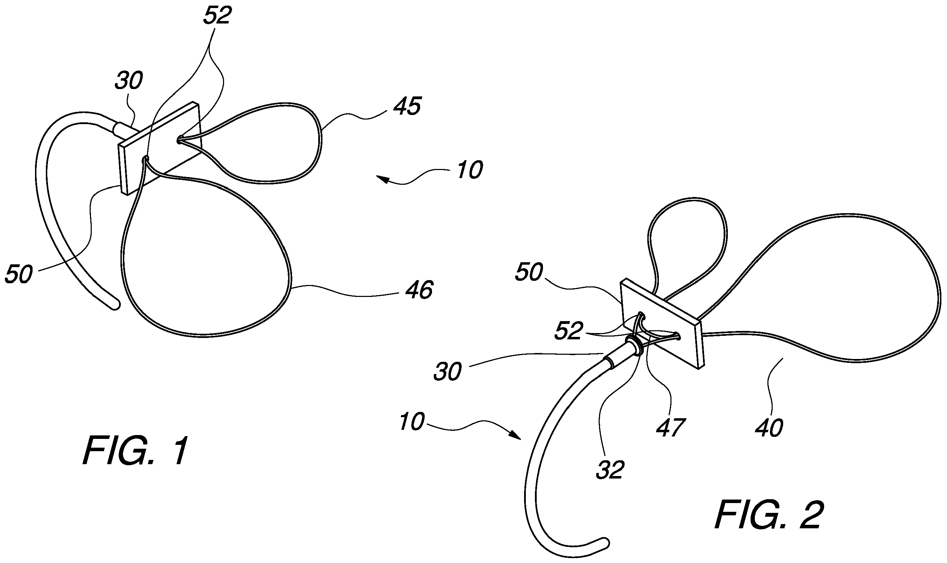

Referring to FIGS. 1 and 2, a pledgetted example is disclosed.

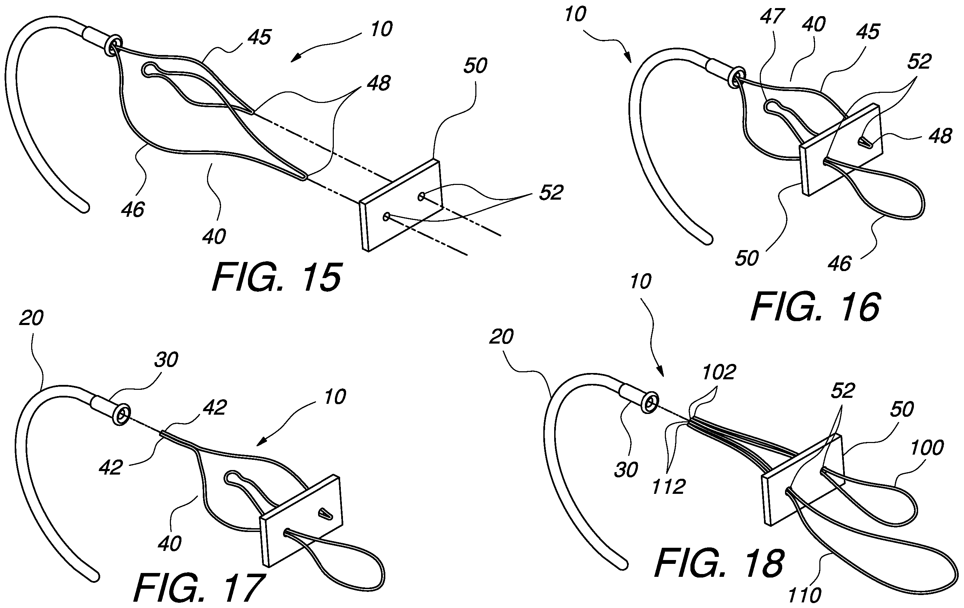

FIG. 1 shows a distal view of a dual snare pledget assembly 10 as presented for use. A large loop 46 and a small loop 45 having passed through and expanded outside of suture holes 52 formed in a pledget 50 in a fashion that allows for the positioning of the pledget 50 in proximity to a suture securing sleeve 30. While FIGS. 1-17 illustrate examples in which the large and small loops are formed from a single length of material, the loops may be formed separately as shown in FIG. 18.

FIG. 2 shows a proximal perspective view of the dual snare pledget assembly 10 of FIG. 1 relative to its intended field of use with the pledget 50 in position close to a head 32 of the suture securing sleeve 30. A folding loop 47 of a wire snare 40 transits the two suture holes 52 of the pledget 50.

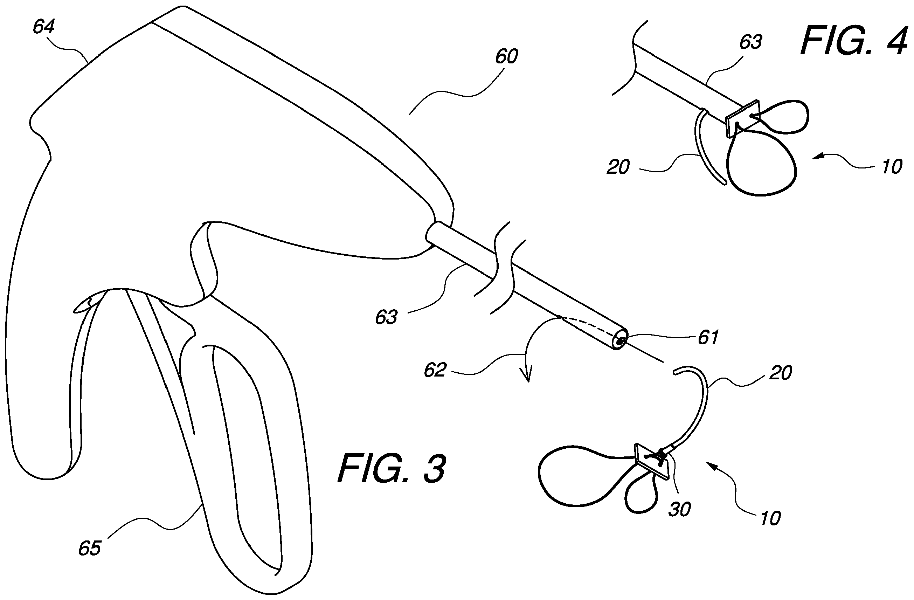

FIG. 3 is a perspective view of the dual snare pledget assembly 10 with its preferred method of application in a mechanical crimping device 60. The mechanical crimping device 60 is preferably a commercially available product such as the Ti-KNOT® Device or COR-KNOT® Device which are marketed exclusively by LSI Solutions, Inc. of Victor, NY. The curved handle 20 of the dual snare pledget assembly 10 is inserted into a sleeve receptacle 61 along an insertion path 62 located at the distal end of a barrel 63 that is fixedly attached to a handle 64 of the mechanical crimping device 60. The mechanical crimping device 60 is mechanically activated via a lever 65 which induces a physically crushing force onto the suture securing sleeve 30 leaving it permanently attached to suture tails emanating from a wound closure site.

Focusing on FIG. 4, the dual snare pledget assembly 10 is shown fully installed and securely seated in the barrel 63 of the mechanical crimping device 60 of FIG. 3. The curved handle 20 is passed through and protrudes from the barrel 63. While a curved handle geometry is preferred to facilitate threading of the handle through the distal end of the mechanical crimping device and to provide greater purchase for pulling on the handle to operate the device during a procedure, a straight handle may also be used, preferably provided with a textured gripping surface.

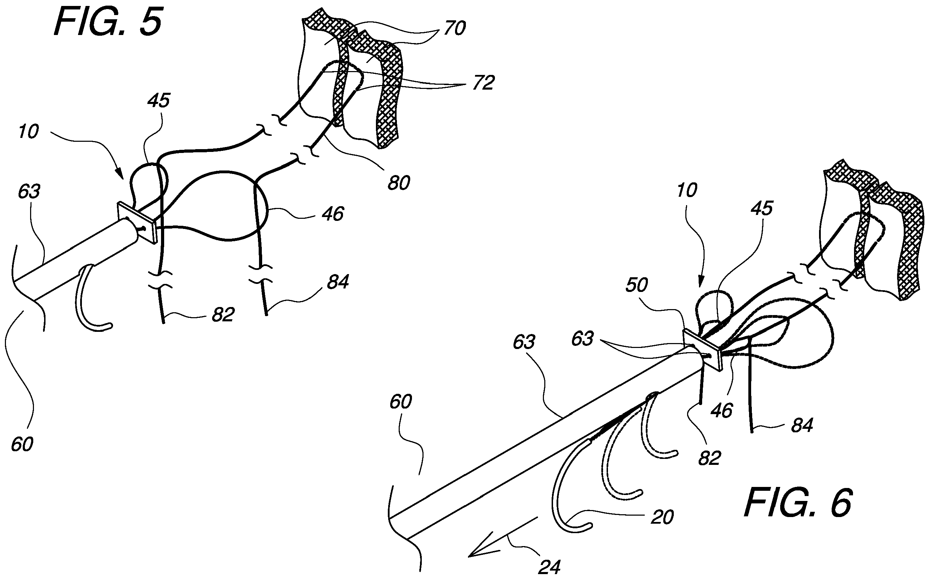

FIGS. 5 through 8 illustrate the sequential threading, passing, and pulling of suture 80 through the dual snare pledget assembly 10, suture securing sleeve 30 and barrel 63 of the mechanical crimping device 60.

FIG. 5 depicts opposing sections of tissue 70 with emplaced suture 80. The suture 80 protrudes from sections of tissue 70 through suture exits 72 such that a left suture tail 82 and a right suture tail 84 remain separated from each other. The left suture tail 82 and the right suture tail 84 are directed towards the mechanical crimping device 60 of FIG. 3 and the installed dual snare pledget assembly 10 with the small loop 45 and large loop 46, respectively. Illustrated is the initial step wherein the left suture tail 82 is threaded through the small loop 45 and kept separated and untangled from the right suture tail 84 which is threaded through the large loop 46. It should be noted, however, that placement of a particular suture tail in a particular loop is not critical so long as the suture 80 is kept untangled, and only one suture tail is fed into each loop.

FIG. 6 shows the sequence of events after FIG. 5 wherein the curved handle 20 is extracted by hand from the barrel 63 of the mechanical crimping device 60 by pulling it in the direction 24. The small loop 45 and large loop 46 are drawn through the suture holes 52 in the pledget 50 and begin the process of passing the left suture tail 82 and right suture tail 84 through the mechanical crimping device 60.

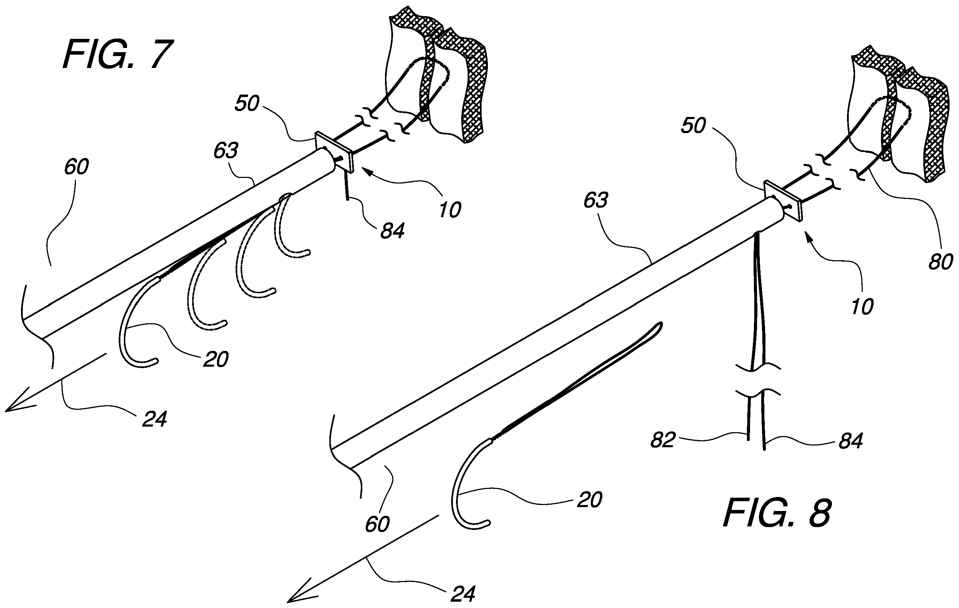

FIG. 7 depicts the sequence of events after FIG. 6 wherein the curved handle 20 is extracted by hand further from the barrel 63 of the mechanical crimping device 60 in the pull direction 24. The previously passed small loop 45 and its threaded left suture tail 82, which are no longer visible, are almost pulled entirely through the barrel 63 and the large loop 46, which is no longer visible, and its threaded right suture tail 84 have passed through the pledget 50 and are being passed through the barrel 63. While small and large loops are preferred to reduce the bulk of the snare as it passes through the mechanical crimping device, loops of the same size may also be used.

FIG. 8 illustrates the final sequence of pulling suture 80 through the pledget 50 and out of the barrel 63 of the mechanical crimping device 60. The curved handle 20 is fully extricated from the barrel 63 in pull direction 24 which subsequently allows for the exit of the left suture tail 82 and the right suture tail 84 from the barrel 63.

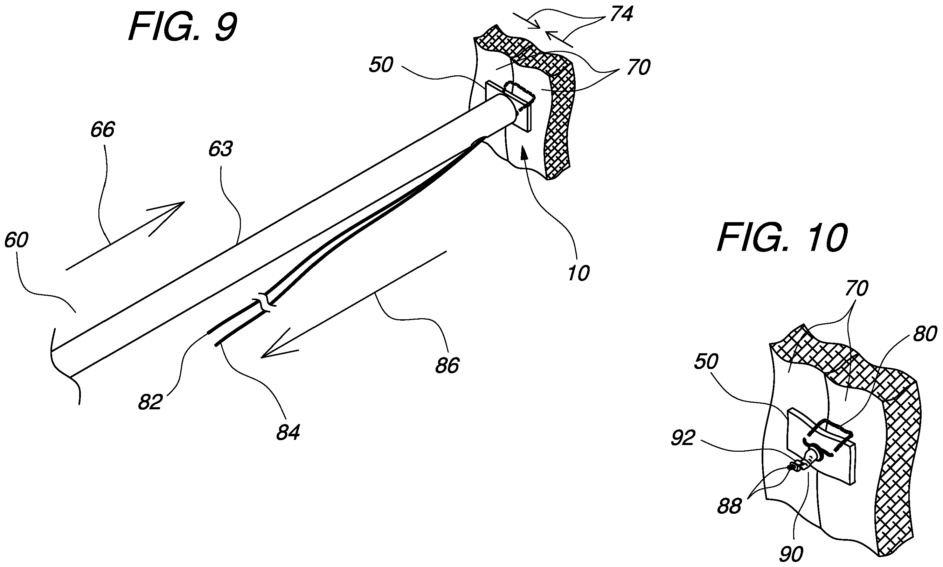

Referring now to FIG. 9, the barrel 63 of the mechanical crimping device 60 is moved along engaging direction 66 and the left suture tail 82 and the right suture tail 84 are pulled taut in direction 86 such that the pledget 50 is cinched against the tissue 70, drawing the opposing sides towards each other in direction 74. Tension is maintained and the mechanical crimping device 60 is actuated to secure the suture with the crimped sleeve 90 (not shown in this view).

FIG. 10 illustrates the effect of the actuated mechanical crimping device 60 as shown in FIG. 9 wherein the pledget 50 is snug to the tissue 70, the suture 80 is taut, and a crimped sleeve 90 is secured against the pledget 50 with trimmed suture 88 exiting a crimped barrel 92.

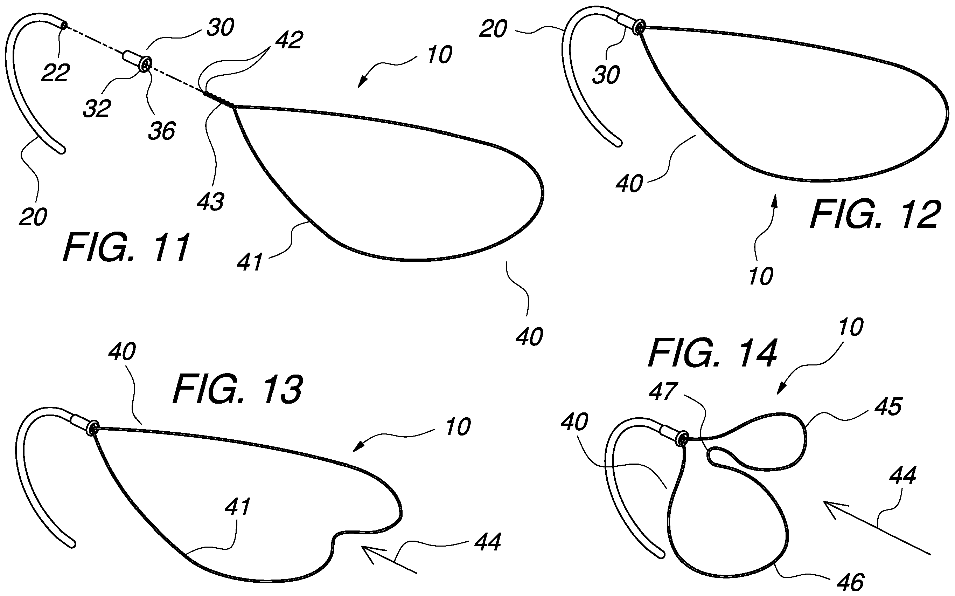

Referring to FIGS. 11 and 12, process steps are shown for a typical assembly of a pledget assembly.

FIG. 11 shows a pledget assembly 10 preferably including a wire or fiber snare 40, a suture securing sleeve 30 having an enlarged head 32, and a curved handle 20. The wire snare 40 is formed of a biocompatible flexible wire such as 304 stainless steel or the like, or a natural or synthetic fiber such as silk or polypropylene or the like, into a loop 41 whose free ends 42 are secured together as a twisted pair end 43. The twisted pair end 43 of wire snare 40 is inserted through a bore 36 of suture securing sleeve 30 such that the head 32 is adjacent to the loop 41.

Traditionally, the suture securing sleeve 30 is made of a medical grade permanently implantable radiopaque material; such as titanium, although absorbable materials such as magnesium can be used. Suitable suture securing sleeves 30 are exclusively marketed by LSI Solutions, Inc., and can be found under commercial trade names Ti-KNOT® and COR-KNOT®. The twisted pair end 43 is inserted into a snare receptacle bore 22 of a curved handle 20 and permanently secured by mechanically crimping the handle. While twisting the ends of the wire snare 40 is preferred, the untwisted ends may, if desired, simply be inserted through the sleeve 30 into the handle 20 and crimped. Typical materials used in the forming of the curved handle 20 include fully hardened stainless steels in the 400 series regime.

FIG. 12 illustrates the assembled dual snare pledget assembly 10 of FIG. 11 before the single snare is formed into two loops. The wire snare 40 passes through the suture securing sleeve 30 and fixedly attached to the curved handle 20.

FIGS. 13 through 16 illustrate the forming sequence of the wire snare 40.

FIG. 13 depicts the dual snare pledget assembly 10 in its raw form with the loop 41 being deformed in a folding direction 44 such that the presentation of a dual loop becomes evident.

FIG. 14 shows a dual snare pledget assembly 10 that, having been formed in folding direction 44, creates a folding loop 47 and two disparate loops of wire; a small loop 45 and a large loop 46.

FIG. 15 demonstrates the additional forming of the wire snare 40 into formed points 48 to facilitate installation through suture holes 52 of the pledget 50. The large loop 46 and the small loop 45 are mechanically pinched to temporarily create formed points 48.

Referring to FIG. 16, the large loop 46 of the wire snare 40 has been passed through one of the suture holes 52 of the pledget 50 and expanded. The small loop 45 with its formed point 48 is shown inserted through and protruding from the opposing suture hole 52 of pledget 50.

FIG. 17 shows an example in which the ends 42 of the wire snare 40 are not twisted or otherwise combined, but fed individually through the suture securing sleeve 30 and into the curved handle 20 where they are mechanically secured, for example, by crimping.

FIG. 18 shows an example wherein free ends 102 of an individual small wire loop 100 and free ends 112 of a large wire loop 110 are separately fed through the suture holes 52 of pledget 50 and subsequently through the suture securing sleeve 30 and into the curved handle 20 where they are mechanically secured.

### Non-Pledgetted Examples

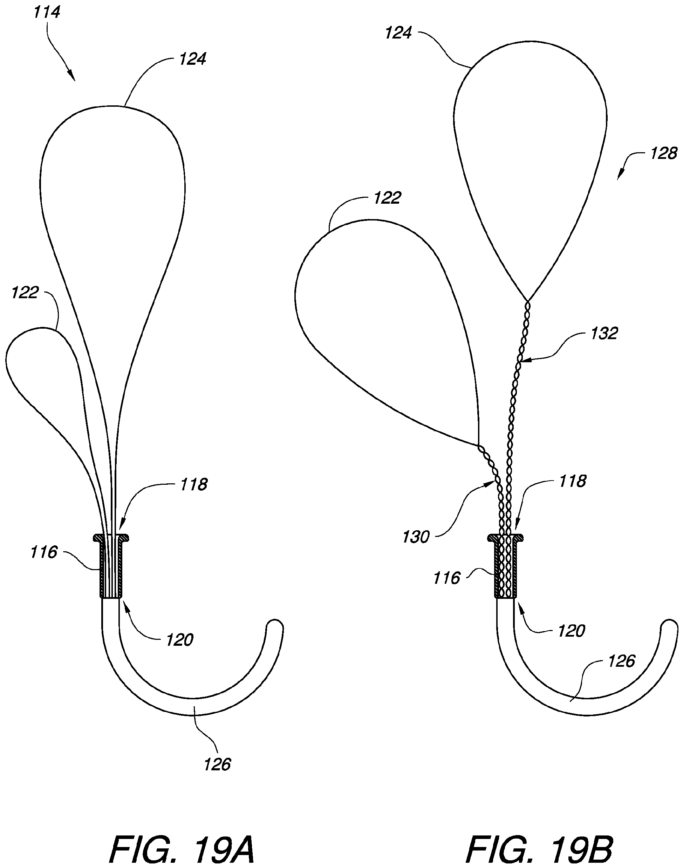

The example of FIG. 18, and the like, can have additional utility without a pledget. As a set of non-exhaustive examples, FIGS. 19A-19J schematically illustrate different examples and embodiments of a surgical snare assembly in partial cross-sectional view. In the case of FIG. 19A, the surgical snare assembly 114 has a suture fastener 116 (shown in cross-sectional view) having an entrance 118 and an exit 120. Some non-limiting examples of a suitable suture fastener include a suture securing sleeve or a mechanical fastener. The surgical snare assembly 114 also has a proximal suture engaging loop 122 and a distal suture engaging loop 124. The distal suture engaging loop 124 extends farther from the handle 126 than the proximal suture engaging loop 122. The suture engaging loops can be made from a wide variety of materials, including, but not limited to, wires, threads, fibers, strings, fabrics, metals, plastics, rubber, or any plurality and/or combination thereof. For convenience, the loop material will often be referred to as a filament.

The surgical snare assembly 114 further has a handle 126. The handle 126 is coupled to the proximal and distal suture engaging loops 122, 124, and configured such that movement of the handle 126 a first distance away from the suture fastener 116 causes the proximal suture engaging loop 122 to move through the suture fastener 116 from the entrance 118 to the exit 120. The handle 126 is also configured such that movement of the handle 126 a second distance away from the suture fastener 116 causes the distal suture engaging loop 124 to move through the suture fastener 116 from the entrance 118 to the exit 120. Although the handle 126 is illustrated herein as being curved, other handle shapes may be used, depending on the example.

The handle 126 can be formed from a variety of materials, including, but not limited to metal, plastic, rubber, or any combination and/or plurality thereof. In the example of FIG. 19A, the handle 126 is directly coupled to both the proximal and distal suture engaging loops 122, 124. In other examples, the handle 126 may be indirectly coupled to either of the proximal or the distal suture engaging loops 122, 124.

The handle 126 and suture fastener 116 are configured to be inserted into a suture fastener applicator (not shown, but non-limiting examples such as the Ti-KNOT® and COR-KNOT® devices have been discussed above). The handle 126 is coupled through the suture fastener 116 to the proximal and distal suture engaging loops 122, 124 such that the suture fastener 116 is slideably and removably held on a portion of at least the proximal suture engaging loop 122 (or a coupling leading thereto) lying between 1) the handle 126 on the exit 120 side of the suture fastener 116 and 2) the proximal and distal suture engaging loops 122, 124 on the entrance 118 side of the suture fastener 116. In the case where the distal suture engaging loop 124 is also directly coupled to the handle 126, the suture fastener 116 will also be slideably and removably held on a portion of the distal suture engaging loop 124 or a coupling leading thereto between 1) the handle 126 on the exit 120 side of the suture fastener 116 and 2) the proximal and distal suture engaging loops 122, 124 on the entrance 118 side of the suture fastener 116.

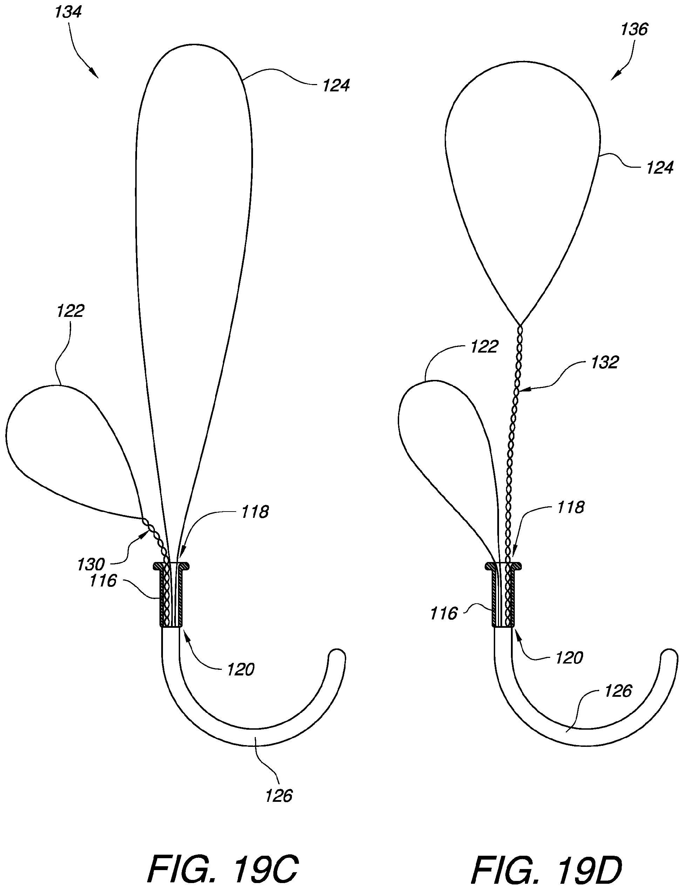

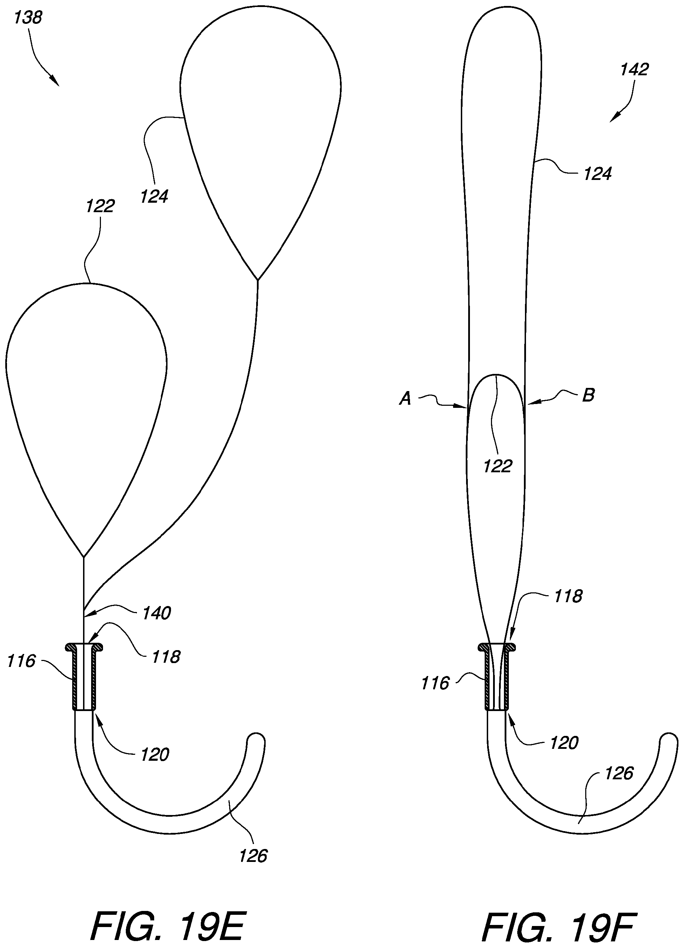

The examples of FIGS. 19B-19E illustrate further non-limiting examples of a suture snare assembly where the distal suture engaging loop is directly coupled to the handle. For example, FIG. 19B illustrates another example of a surgical snare assembly 128. In FIG. 19B, the proximal suture engaging loop 122 is directly coupled to the handle 126 by a proximal twisted filament 130. A twisted filament can be one or more filaments twisted together. Likewise, the distal suture engaging loop 124 is directly coupled to the handle 126 by a distal twisted filament 132. FIGS. 19C and 19D illustrate further embodiments of a surgical snare assembly 134, 136, respectively. In FIG. 19C, the proximal suture engaging loop 122 is directly coupled to the handle 126 by a proximal twisted filament 130, while the distal loop 124 is directly coupled to the handle 126. In FIG. 19D, the distal suture engaging loop 124 is directly coupled to the handle 126 by a distal twisted filament 132, while the proximal loop 122 is directly coupled to the handle 126. FIG. 19E illustrates another embodiment of a surgical snare assembly 138. In FIG. 19E, the proximal and distal suture engaging loops 122, 124 are directly coupled to the handle 126 by a branched filament 140.

As with the example of FIG. 19A, the examples of FIGS. 19B-19E also have a suture fastener 116 (shown in cross-sectional view) having an entrance 118 and an exit 120. The handle 126 is still configured such that movement of the handle 126 a first distance away from the suture fastener 116 causes the proximal suture engaging loop 122 to move through the suture fastener 116 from the entrance 118 to the exit 120. The handle 126 is also configured such that movement of the handle 126 a second distance away from the suture fastener 116 causes the distal suture engaging loop 124 to move through the suture fastener 116 from the entrance 118 to the exit 120.

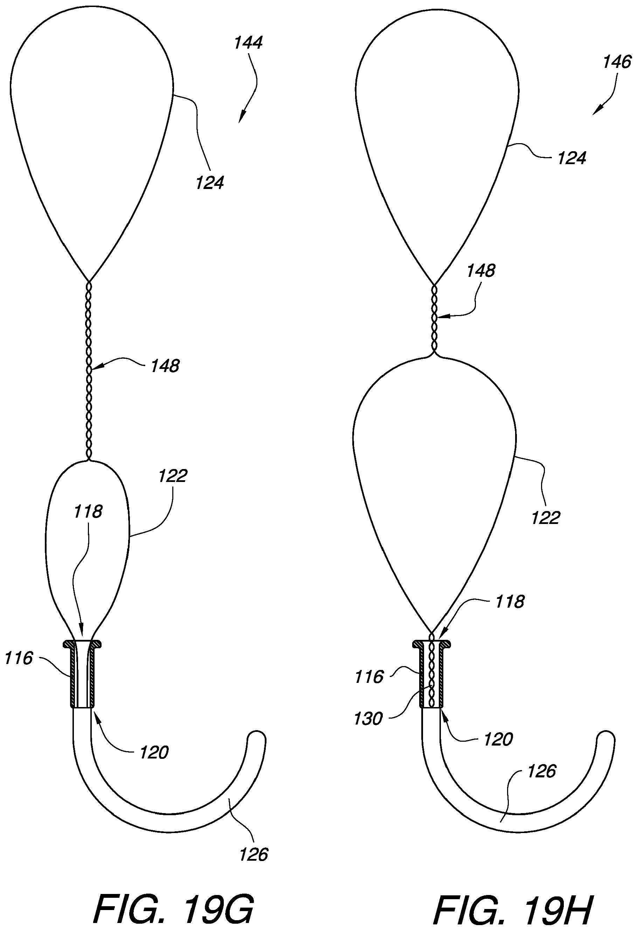

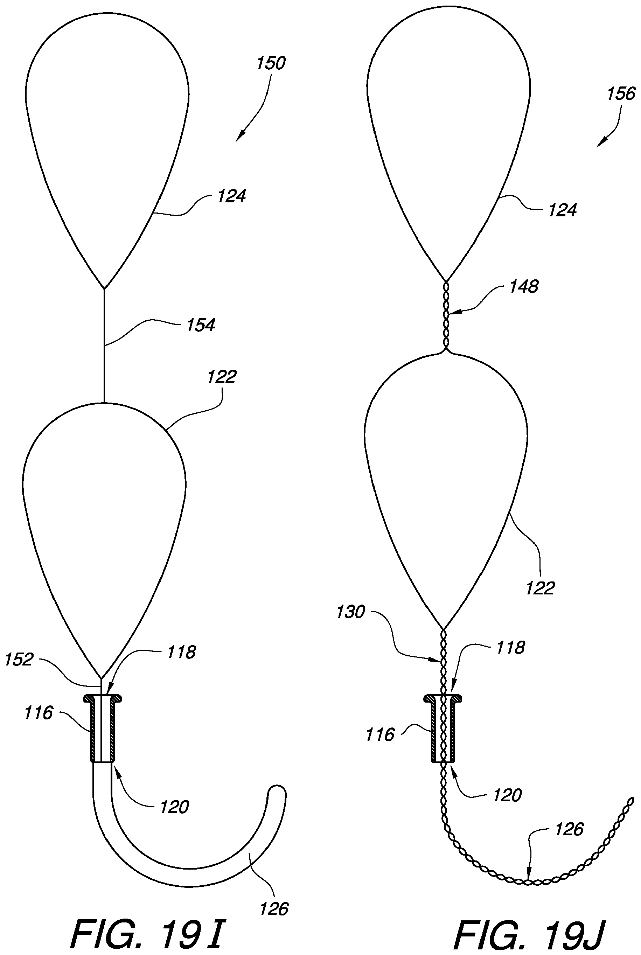

The examples and embodiments of FIGS. 19F-19I illustrate non-limiting examples of a suture snare assembly where the distal suture engaging loop is indirectly coupled to the handle. For example, FIG. 19F illustrates an example of a surgical snare assembly 142 where the proximal loop 122 is directly coupled to the handle 126 and the distal loop 124 is indirectly coupled to the handle 126 via the proximal loop 122. In this example, the distal loop 124 is attached to the proximal loop 122 at points A and B. Arguably, one could say that the proximal loop 122 is indirectly coupled to the handle 126 by the distal loop 124 at the same points A, B, however, the convention used herein is to say the proximal loop 122 is the directly coupled loop when the interpretation could go either way. FIGS. 19G and 19H illustrate further embodiments of a surgical snare assembly 144, 146, respectively. In FIG. 19G, the proximal suture engaging loop 122 is directly coupled to the handle 126, while the distal suture engaging loop 124 is indirectly coupled to the handle 126 by the proximal suture engaging loop 122 and a twisted filament 148 extending between the proximal suture engaging loop 122 and the distal suture engaging loop 124. In FIG. 19H, the proximal suture engaging loop 122 is directly coupled to the handle 126 by a proximal twisted filament 130, while the distal suture engaging loop 124 is indirectly coupled to the handle 126 by the proximal twisted filament 130, the proximal suture engaging loop 122, and a twisted filament 148 extending between the proximal suture engaging loop 122 and the distal suture engaging loop 124. FIG. 19I illustrates another embodiment of a surgical snare assembly 150. In FIG. 19I, the proximal suture engaging loop 122 is directly coupled to the handle 126 by a first filament 152, while the distal suture engaging loop 124 is indirectly coupled to the handle 126 by the first filament 152, the proximal suture engaging loop 122, and a second filament 154 extending between the proximal suture engaging loop 122 and the distal suture engaging loop 124. In general, the distal suture engaging loop will be indirectly coupled to the handle if the path from the distal loop to the handle includes the proximal loop.

As with the example of FIG. 19A, the examples and embodiments of FIGS. 19F-19I also have a suture fastener 116 (shown in cross-sectional view) having an entrance 118 and an exit 120. The handle 126 is still configured such that movement of the handle 126 a first distance away from the suture fastener 116 causes the proximal suture engaging loop 122 to move through the suture fastener 116 from the entrance 118 to the exit 120. The handle 126 is also configured such that movement of the handle 126 a second distance away from the suture fastener 116 causes the distal suture engaging loop 124 to move through the suture fastener 116 from the entrance 118 to the exit 120.

FIG. 19J illustrates another embodiment of a surgical snare assembly 156. The embodiment of FIG. 19J is similar to the embodiment of FIG. 19H, discussed above, however, the handle 126 in FIG. 19J comprises a material which extends to form the proximal and distal suture engaging loops 122, 124, rather than having a separate item for the loops which would have to be attached to the handle. Such an embodiment could be formed, for example, from a single filament that is first looped to form the distal suture engaging loop 124, then twisted to form a twisted filament segment 148, then looped again to form the proximal suture engaging loop 122, and then twisted again to form a proximal twisted filament segment 130 that passes through a suture fastener 116 and is then curved or otherwise shaped or left hanging out of the suture fastener 116 as a handle 126.

As with the example of FIG. 19A, the suture fastener 116 in FIG. 19J also has an entrance 118 and an exit 120. The handle 126 is still configured such that movement of the handle 126 a first distance away from the suture fastener 116 causes the proximal suture engaging loop 122 to move through the suture fastener 116 from the entrance 118 to the exit 120. The handle 126 is also configured such that movement of the handle 126 a second distance away from the suture fastener 116 causes the distal suture engaging loop 124 to move through the suture fastener 116 from the entrance 118 to the exit 120.

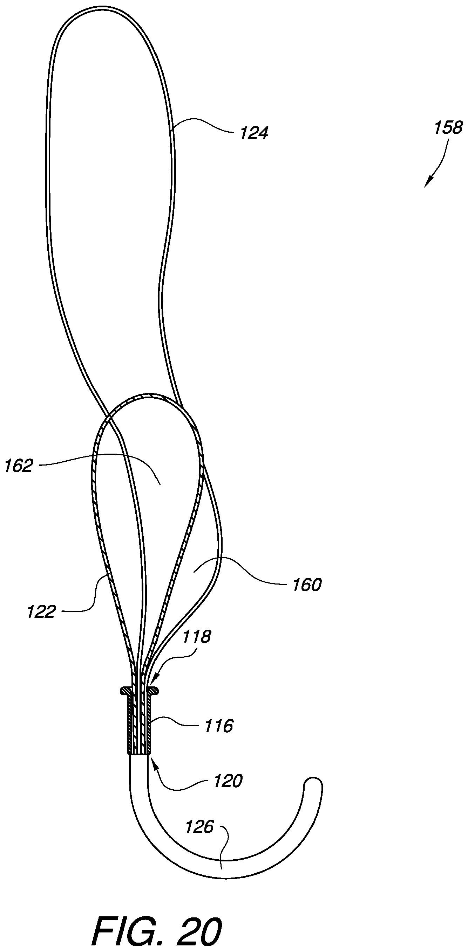

As illustrated with the surgical snare assembly 158 of FIG. 20, and depending on the example, it is sometimes possible that a portion of a distal suture engaging loop 124 will overlap at least a portion of a proximal suture engaging loop 122. If the filament used to form the proximal and distal suture engaging loops 122, 124 is similar or identical, then the overlap may make it more difficult to differentiate the loops 122, 124 in such a way that a suture can reliably be placed into the proximal loop 122. For example, and with reference to FIG. 20, if the proximal suture engaging loop 122 material looked identical to the suture engaging loop material (which is not the case here), then first target area 160 could easily be mistaken as being in the proximal loop 122 when in fact it is part of the distal loop 124. With the example of FIG. 20, however, the proximal suture engaging loop 122 is differentiable from the distal suture engaging loop 1 24, thereby helping to ensure surgeons and surgical staff can easily tell one overlapping loop from another. In this case, based on the loop differentiation, a second target area 162 is easily identifiable as being in the proximal suture engaging loop 122. Depending on the example, the loops can be differentiable by a variety of characteristics including, but not limited to, color, texture, material type, and material dimension.

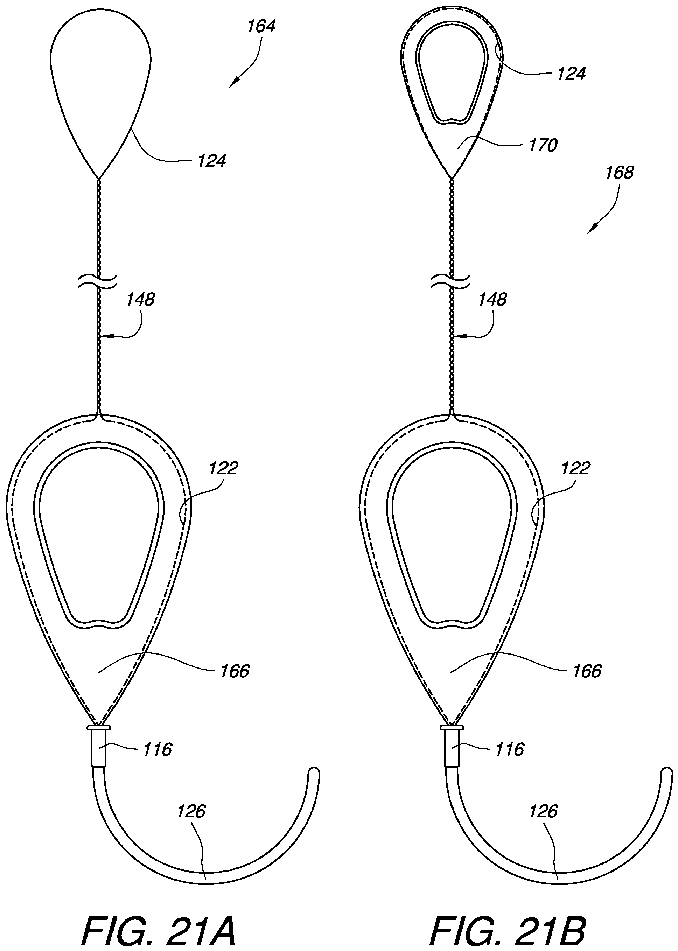

Since the proximal and distal suture engaging loops may be delicate structures in some embodiments, it may be desirable to provide support for one or more of the loops when packaging the surgical snare assemblies. As one example, FIG. 21A illustrates one embodiment of a surgical snare assembly 164, similar to the surgical snare assembly of FIG. 19H discussed previously. However, the embodiment of FIG. 21A also provides a proximal holder 166 to support the proximal suture engaging loop 122. The proximal holder 166 may be configured so the proximal suture engaging loop 122 can be removed from the holder 166 as desired when the assembly 164 is being used. Proximal holder 166 also provides support to hold the suture fastener 116 in place against the handle 126 until the proximal holder 166 is removed. Another advantage of the proximal holder 166 is that it helps to prevent premature pulling of the proximal loop 1 22 through the suture fastener 116. In the embodiment of FIG. 21A, the distal suture engaging loop 124 is unsupported.

FIG. 21B illustrates one embodiment of a surgical snare assembly 168 having a proximal holder 166 for the proximal suture engaging loop 122 and a distal holder 170 for the distal suture engaging loop 124. The distal holder 170 may be configured, similarly to the proximal holder 166, such that the distal suture engaging loop 124 can be removed from the distal holder 170 when desired.

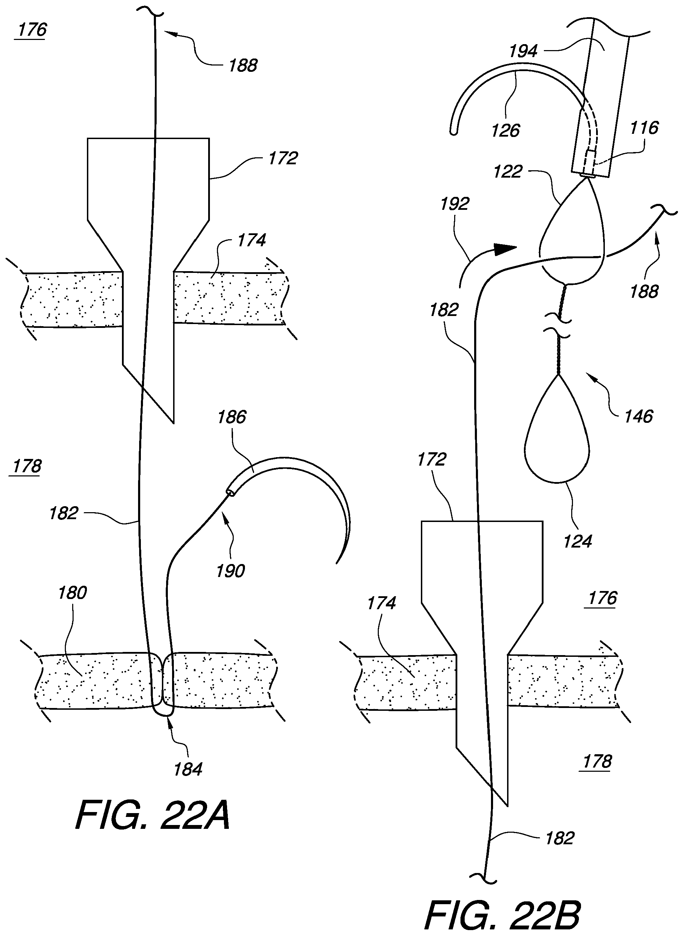

The surgical snare embodiments discussed herein, and their equivalents, are very useful in minimally invasive surgical (MIS) procedures. For example, FIGS. 22A-22G illustrate one embodiment of a surgical snare assembly in use with a suture fastener applicator to enable internal tensioning of a suture stitch for suture fastener application while an assistant anchors one end of the suture externally. FIG. 22A schematically illustrates a surgical situation. One or more cannulas 172 have been placed through incisions in a patient's outer tissue layers 174, such as, but not limited to a thoracic wall, providing access from an area 176 outside the patient's body to an area 178 inside the patient's body, such as, but not limited to, a thoracic cavity. For convenience, only one cannula 172 is shown. In the example of FIG. 22A, the internal area 178 has some internal tissue 180 which has been stitched at least once by a suture 182. The at least one suture stitch 184 may have been placed using a needle 186 manipulated through the one or more cannulas 172. Other examples may use specialized MIS suturing devices, such as, but not limited to the RD180® running stitch suturing device available from LSI Solutions, Inc. of Victor, NY. It should be understood that the term "suture", as used herein, is intended to cover any thread, cable, wire, filament, strand, line, yarn, gut, or similar structure, whether natural and/or synthetic, in monofilament, composite filament, or multifilament form (whether braided, woven, twisted, or otherwise held together), as well as equivalents, substitutions, combinations, and pluralities thereof for such materials and structures.

One end 188 of the suture 182 may be kept outside of the patient, while another end 190 of the suture 182 is inside the patient. If a surgeon is manipulating the instruments (not shown) necessary to operate inside the patient, an assistant can place 192 the one suture end 188 through a proximal suture engaging loop 122 of a surgical snare assembly, such as the surgical snare assembly 146 discussed above and as shown in FIG. 22B. The handle 126 of the surgical snare assembly 146 is configured in this example to guide the suture fastener 116 into a suture fastener applicator 194. The suture fastener applicator 194 has the ability to crimp, deform, or otherwise attach or apply the suture fastener 116 to one or more sutures passed therethrough. Suitable non-limiting examples of a suture fastener applicator 194 include the mechanical crimping device 60 discussed previously with regard to FIGS. 3-9, such as the Ti-KNOT® Device or COR-KNOT® Device which are marketed exclusively by LSI Solutions, Inc. of Victor, NY. Preferably, the surgical snare assembly 146 will be installed into the suture fastener applicator 194 (as shown in FIG. 22B) before the one suture end 188 is placed 192 through the proximal suture engaging loop 122, although that order of actions could be reversed or made simultaneous if desired.

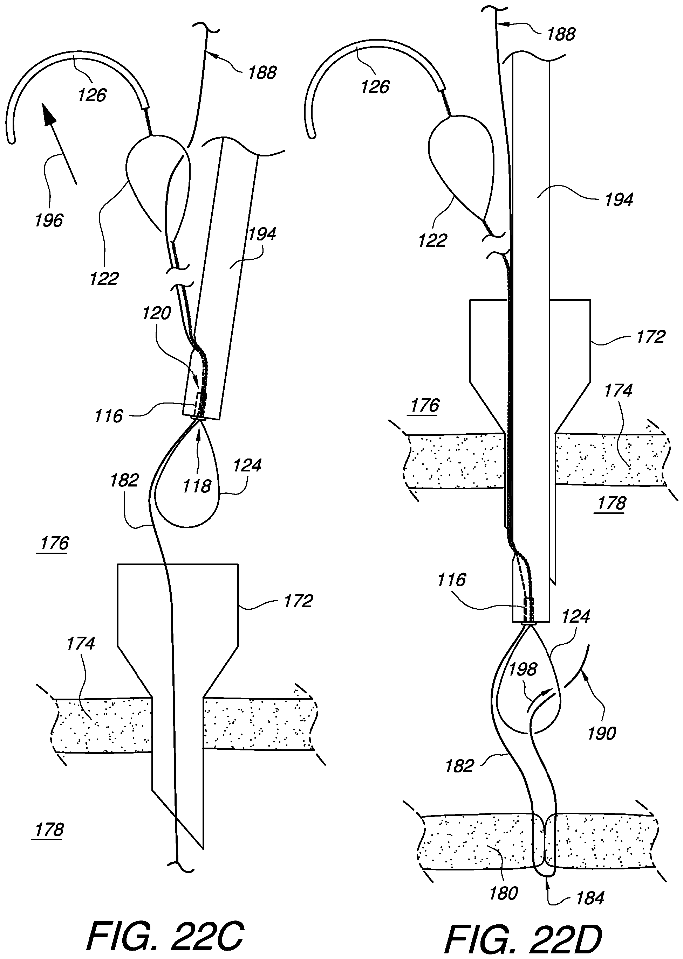

As illustrated in FIG. 22C, the handle 126 has been moved 196 away from the suture fastener 116 far enough to cause the proximal suture engaging loop 122, and consequently the one suture end 188 snared in that loop 122, to move through the suture fastener 116 from an entrance 118 to an exit 120 of the suture fastener 116. This first movement 196 of the handle 126 is not enough to pull the distal suture engaging loop 124 all the way through the suture fastener 116. Accordingly, the distal suture engaging loop 124 is still coupled through the suture fastener 116 to the handle 126.

The suture fastener applicator 194 can then be used to put the distal suture engaging loop 124 through the cannula 172 and into the area 178 inside the patient's body as shown in FIG. 22D. If desired, this can be accomplished by a surgical assistant who can also hold the one suture end 188 in the area 176 outside the body. As shown in FIG. 22D, the one suture end 188 can be removed from the proximal suture engaging loop 122 once the suture end 188 has been pulled through the suture fastener 116. In the area 178 inside the body, the other end 190 of the suture 182 can be placed 198 through the distal suture engaging loop 124 while the distal suture engaging loop 124 is coupled through the suture fastener 116 to the handle 126.

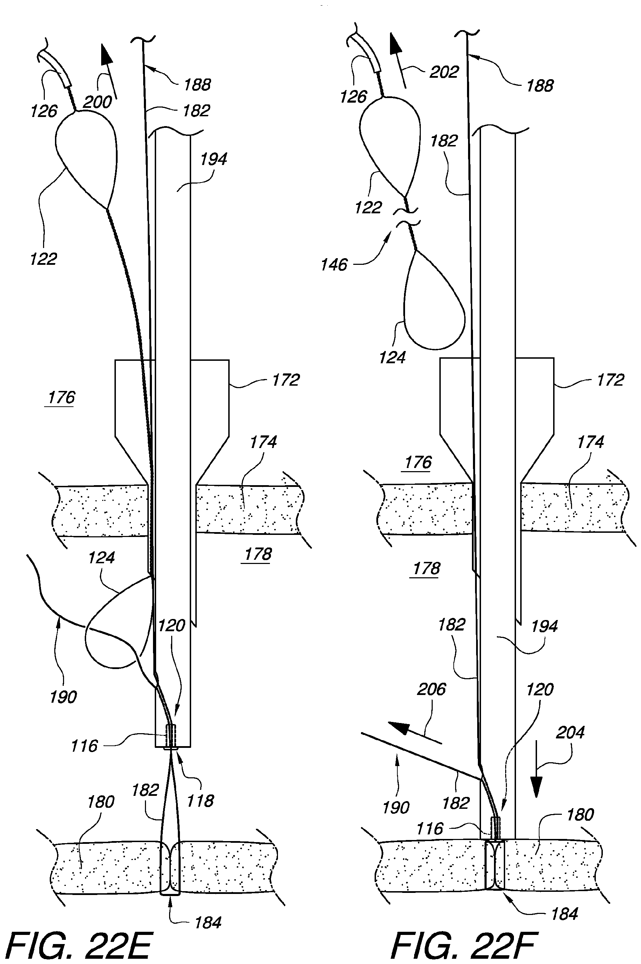

As illustrated in FIG. 22E, the handle 126 has been moved 200 away from the suture fastener 116 far enough to cause the distal suture engaging loop 124, and consequently the other suture end 190 snared in that loop 124, to move through the suture fastener 116 from an entrance 118 to an exit 120 of the suture fastener 116. This second movement 200 of the handle 126 is enough to pull the distal suture engaging loop 124 through the suture fastener 116, but not enough to pull the distal loop 124 out of the patient. Accordingly, the other end 190 of the suture 182 (which has just been pulled through fastener 116) remains available inside 178 the body.

After the other end 190 of the suture 182 has been pulled by the distal suture engaging loop 124 through the suture fastener 116, the suture end 190 may be removed from the loop 124 internally by the surgeon using MIS surgical tools at his or her disposal, whether manually or robotically operated. As illustrated in FIG. 22F, the surgical snare assembly 146 can be pulled 202 out of the cannula 172, while the first end 188 of the suture 182 is anchored or held by a surgical assistant outside 176 the body. The suture fastener applicator 194 can be moved 204 towards the stitch site 184, and the surgeon can internally tension 206 the second suture end 190 to remove slack in the suture 182 and position the suture fastener 116 into a place where the suture stitch 184 can be held properly. It is very important that the suture tension is not too light, or the stitch 184 may have too much play. Similarly, it is important that the suture tension is not too strong in order to avoid tissue ischemia. Using the surgical suture snare and methods described herein, the surgeon can control the tension internally, even when operating through an MIS robotic interface, while a surgical assistant merely anchors or holds the other end of the suture outside the patient. Such methods, and their equivalents obviate the need for a second surgeon to assist with suture tensioning. In other examples, the internal end 190 of the suture could be anchored, while the external end 188 could be tensioned.

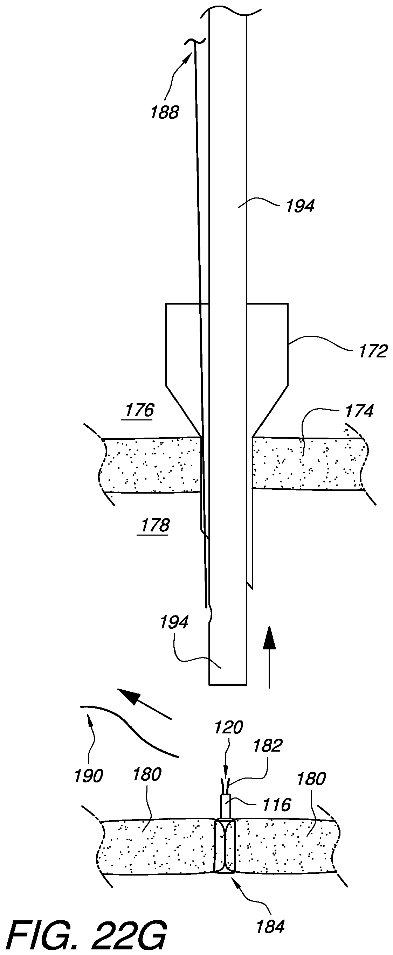

When the surgeon determines that the tension is correct, the surgeon or the surgical assistant can activate the suture fastener applicator 194 to attach the suture fastener 116 to the one or more sutures 182 therein. The suture fastener applicator 194 may also be configured to cut the sutures on the exit 120 side of the suture fastener 116. Optionally, the suture 182 may be similarly cut with a separate MIS tool. The result is illustrated in FIG. 22G, where the suture fastener 116 is in place, successfully attached to suture 182 to hold stitch 184 in place. The first suture end 188, now cut loose, can be removed by the assistant anchoring it outside 176 the body. The second suture end 190, also cut loose, can be removed by the surgeon (for example, with an MIS tool), and the suture fastener applicator 194 may be removed from the patient's body.

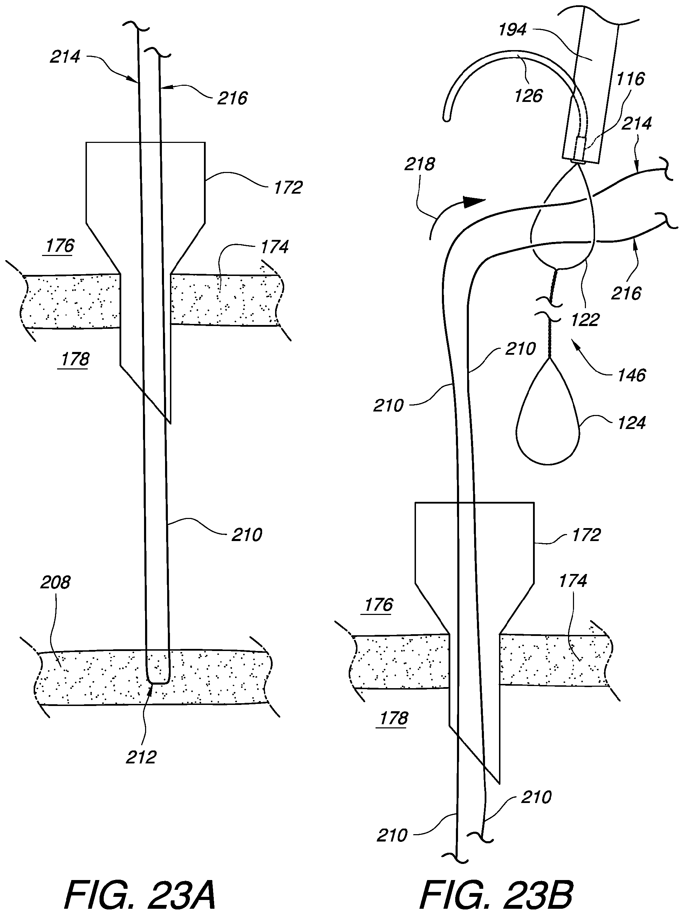

FIGS. 23A-23G illustrate one embodiment of a surgical snare assembly in use with a suture fastener applicator to enable internal threading of one end of a second suture through a mechanical fastener being applied internally to a first suture's stitch whose first and second ends are held externally. FIG. 23A schematically illustrates a surgical situation. One or more cannulas 172 have been placed through incisions in a patient's outer tissue layers 174 as discussed with previous examples, providing access from an area 176 outside the patient's body to an area 178 inside the patient's body. In the example of FIG. 23A, the internal area 178 has some internal tissue 208 which has been stitched at least once by a first suture 210. The at least one suture stitch 212 may have been placed using any of a number of MIS techniques known to those skilled in the art, such as, but not limited to the examples discussed previously and their equivalents.

First and second ends 214, 216 of the first suture 210 are kept outside 176 of the patient. If a surgeon is manipulating the instruments (not shown) necessary to operate inside 178 the patient, an assistant can place 218 both the first and second ends 214, 216 of the first suture 210 through a proximal suture engaging loop 122 of a surgical snare assembly, such as the surgical snare assembly 146 discussed above and as shown in FIG. 23B. The handle 126 of the surgical snare assembly 146 is configured in this example to guide the suture fastener 116 into a suture fastener applicator 194, the details of which have been discussed above. Preferably, the surgical snare assembly 146 will be installed into the suture fastener applicator 194 (as shown in FIG. 23B) before the first and second suture ends 214, 216 of the first suture 210 are placed 218 through the proximal suture engaging loop 122, although that order of actions could be reversed or made simultaneous if desired.

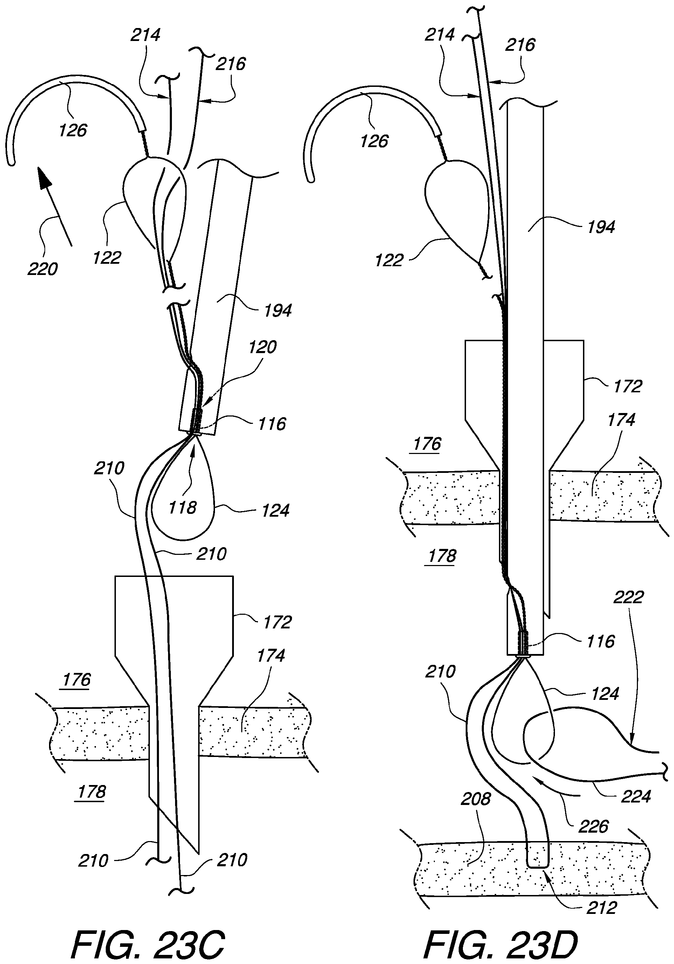

As illustrated in FIG. 23C, the handle 126 has been moved 220 away from the suture fastener 116 far enough to cause the proximal suture engaging loop 122, and consequently the first and second suture ends 214, 216 snared in that loop 122, to move through the suture fastener 116 from an entrance 118 to an exit 120 of the suture fastener 116. This first movement 220 of the handle 126 is not enough to pull the distal suture engaging loop 124 all the way through the suture fastener 116. Accordingly, the distal suture engaging loop 124 is still coupled through the suture fastener 116 to the handle 126

The suture fastener applicator 194 can then be used to put the distal suture engaging loop 124 through the cannula 172 and into the area 178 inside the patient's body as shown in FIG. 23D. If desired, this can be accomplished by a surgical assistant who can also hold the first and second suture ends 214, 216 of the first suture 210 in the area 176 outside the body. As shown in FIG. 23D, the first and second suture ends 214, 216 of the first suture 210 can be removed from the proximal suture engaging loop 122 once the suture ends 214, 216 have been pulled through the suture fastener 116. In the area 178 inside the body, a first end 222 of a second suture 224 can be placed 226 through the distal suture engaging loop 124 while the distal suture engaging loop 124 is coupled through the suture fastener 116 to the handle 126.

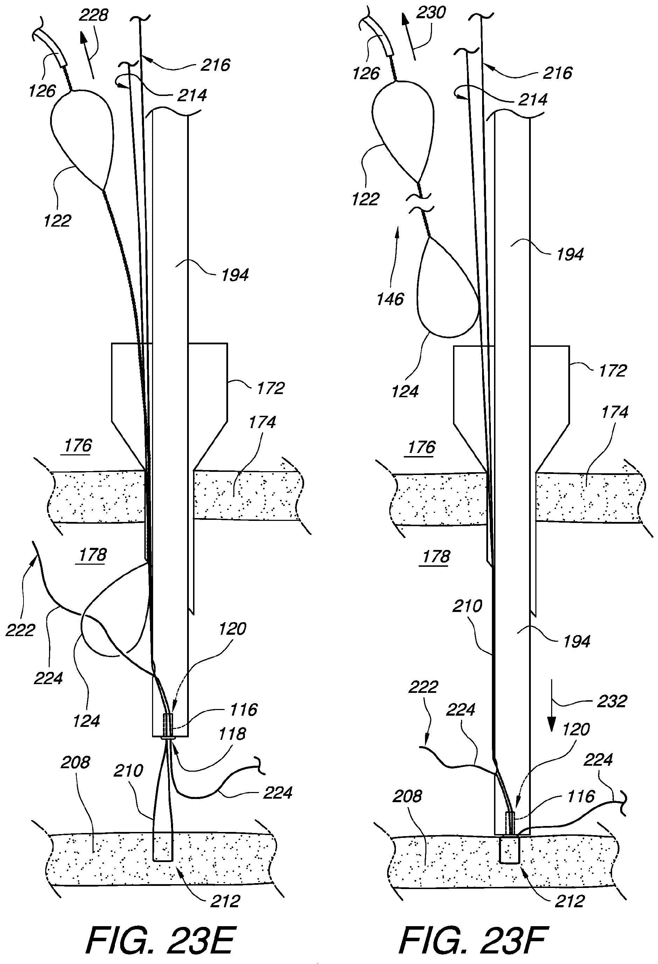

As illustrated in FIG. 23E, the handle 126 has been moved 228 away from the suture fastener 116 far enough to cause the distal suture engaging loop 124, and consequently the first suture end 222 of the second suture 224 snared in that loop 124, to move through the suture fastener 116 from an entrance 118 to an exit 120 of the suture fastener 116. This second movement 228 of the handle 126 is enough to pull the distal suture engaging loop 124 through the suture fastener 116, but not enough to pull the distal loop 124 out of the patient. Accordingly, the first end 222 of the second suture 224 (which has just been pulled through the fastener 116) remains available inside 178 the body.

After the first end 222 of the second suture 224 has been pulled by the distal suture engaging loop 124 through the suture fastener 116, the suture end 222 may be removed from the loop 124 internally by the surgeon with MIS surgical tools at his or her disposal, whether manually or robotically operated. As illustrated in FIG. 23F, the surgical snare assembly 146 can be pulled 230 out of the cannula 172, while the first and second ends 214, 216 of the first suture 210 are tensioned outside 176 the body. The suture fastener applicator 194 can be moved 232 towards the stitch 212 in the internal tissue 208, and the surgeon may hold the first suture end 222 of the second suture 224 off to the side (but not necessarily under tension)

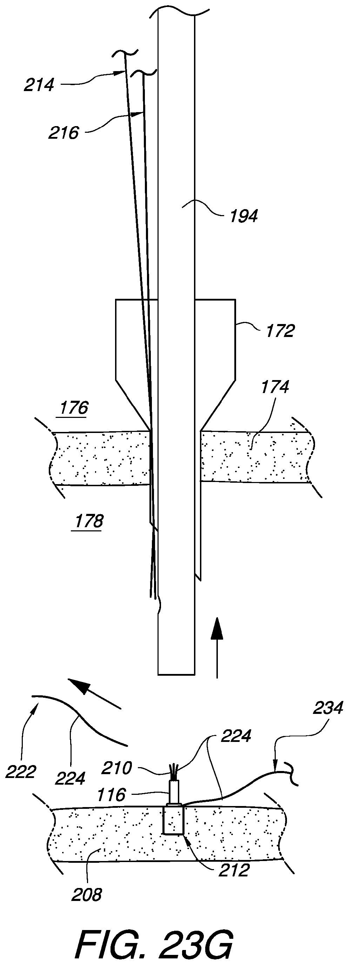

When the surgeon determines that the tension in at least the first suture 210 is correct, the surgeon or the surgical assistant can activate the suture fastener applicator 194 to attach the suture fastener 116 to the one or more sutures 210, 224 therein. The suture fastener applicator 194 may also be configured to cut the sutures on the exit 120 side of the suture fastener 116. Optionally, the sutures 210, 224 may be similarly cut with a separate MIS tool. The result is illustrated in FIG. 23G, where the suture fastener 116 is in place, successfully attached 1) to suture 210 to hold stitch 212 in place, and 2) to suture 224 to provide an anchored end for the free end 234 of the second suture 224. The former first and second ends 214, 216 of the first suture 210, now cut loose, can be removed by someone outside 176 the body. The first end 222 of the second suture 224, also cut loose, can be removed by the surgeon using an MIS tool, and the suture fastener applicator 194 may be removed from the patient's body.

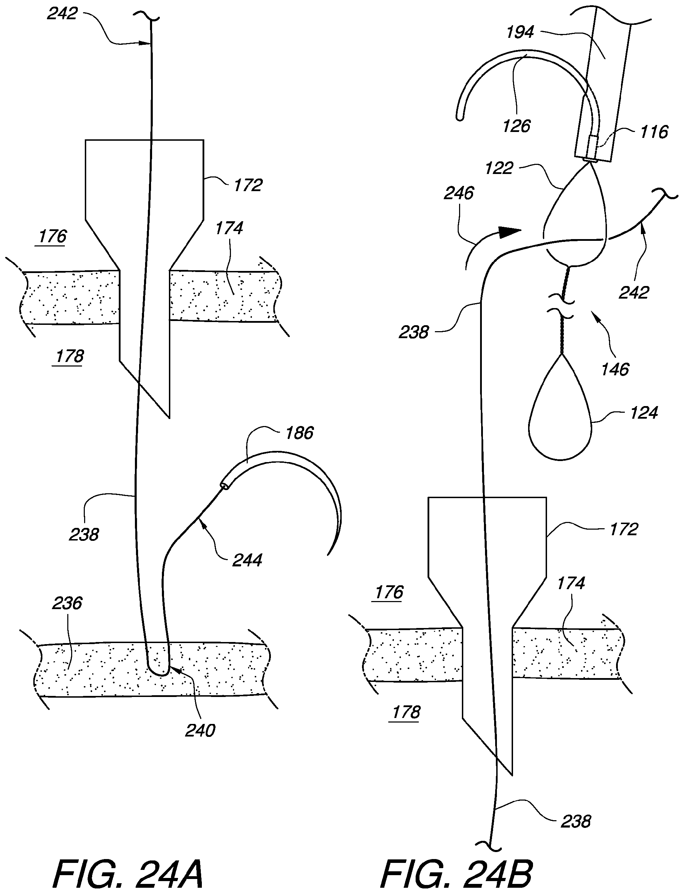

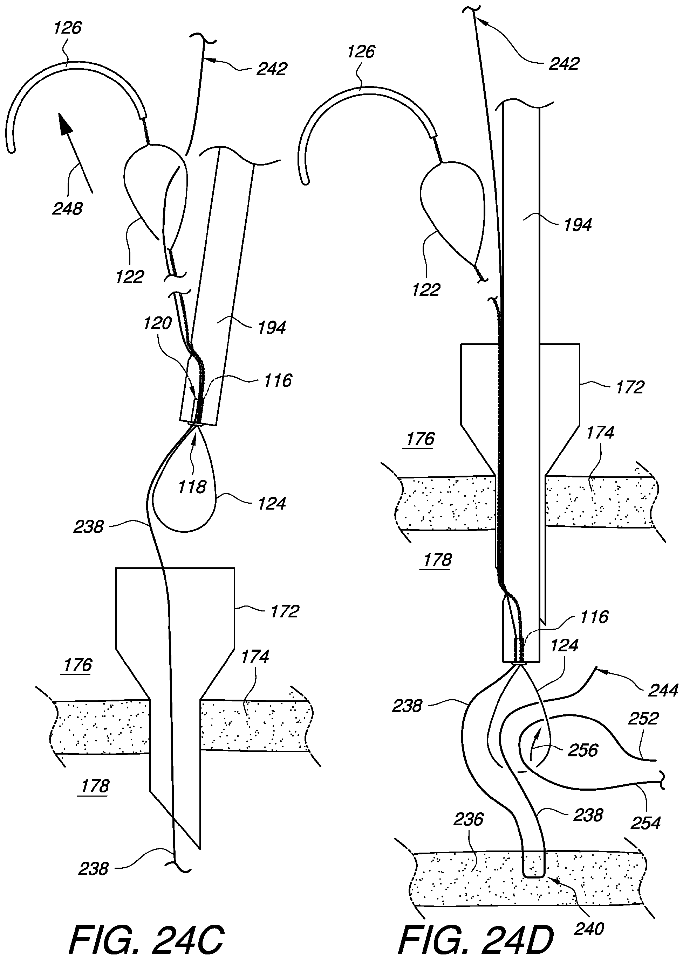

FIGS. 24A-24G illustrate one embodiment of a surgical snare assembly in use with a suture fastener applicator to enable internal tensioning of a suture stitch from a first suture for suture fastener application while an assistant anchors a second end of the first suture externally, and while enabling internal threading of one end of a second suture through a suture fastener being applied to the first suture. FIG. 24A schematically illustrates a surgical situation. One or more cannulas 172 have been placed through incisions in a patient's outer tissue layers 174, as discussed with previous examples, providing access from an area 176 outside the patient's body to an area 178 inside the patient's body. In the example of FIG. 24A, the internal area 178 has some internal tissue 236 which has been stitched at least once by a first suture 238. The at least one suture stitch 240 may have been placed using any of a number of MIS techniques known to those skilled in the art, such as, but not limited to the examples discussed previously and their equivalents.

One end 242 of the first suture 238 may be kept outside of the patient, while another end 244 of the first suture 238 is inside the patient. While a surgeon is manipulating the instruments (not shown) necessary to operate inside the patient, an assistant can place 246 the first suture end 242 of the first suture 238 through a proximal suture engaging loop 122 of a surgical snare assembly, such as the surgical snare assembly 146 discussed above and as shown in FIG. 24B. The handle 126 of the surgical snare assembly 146 is configured in this example to guide the suture fastener 116 into a suture fastener applicator 194, the details of which have been discussed above. Preferably, the surgical snare assembly 146 will be installed into the suture fastener applicator 194 (as shown in FIG. 24B) before the first end 242 of the first suture 238 is placed 246 through the proximal suture engaging loop 122, although that order of actions could be reversed or made simultaneous if desired.

As illustrated in FIG. 23C, the handle 126 has been moved 248 away from the suture fastener 116 far enough to cause the proximal suture engaging loop 122, and consequently the first suture's first end 242 snared in that loop 122, to move through the suture fastener 116 from an entrance 118 to an exit 120 of the suture fastener 116. This first movement 248 of the handle 126 is not enough to pull the distal suture engaging loop 124 all the way through the suture fastener 116. Accordingly, the distal suture engaging loop 124 is still coupled through the suture fastener 116 to the handle 126.

The suture fastener applicator 194 can then be used to put the distal suture engaging loop 124 through the cannula 172 and into the area 178 inside the patient's body as shown in FIG. 24D. If desired, this can be accomplished by a surgical assistant who can also hold the first end 242 of the first suture 238 in the area 176 outside the body. As shown in FIG. 24D, the first end 242 of the first suture 238 can be removed from the proximal suture engaging loop 122 once the suture end 242 has been pulled through the suture fastener 116. In the area 178 inside the body, the second end 244 of the first suture 238 and a first end 252 of a second suture 254 can be placed 256 through the distal suture engaging loop 124 while the distal suture engaging loop 124 is coupled through the suture fastener 116 to the handle 126.

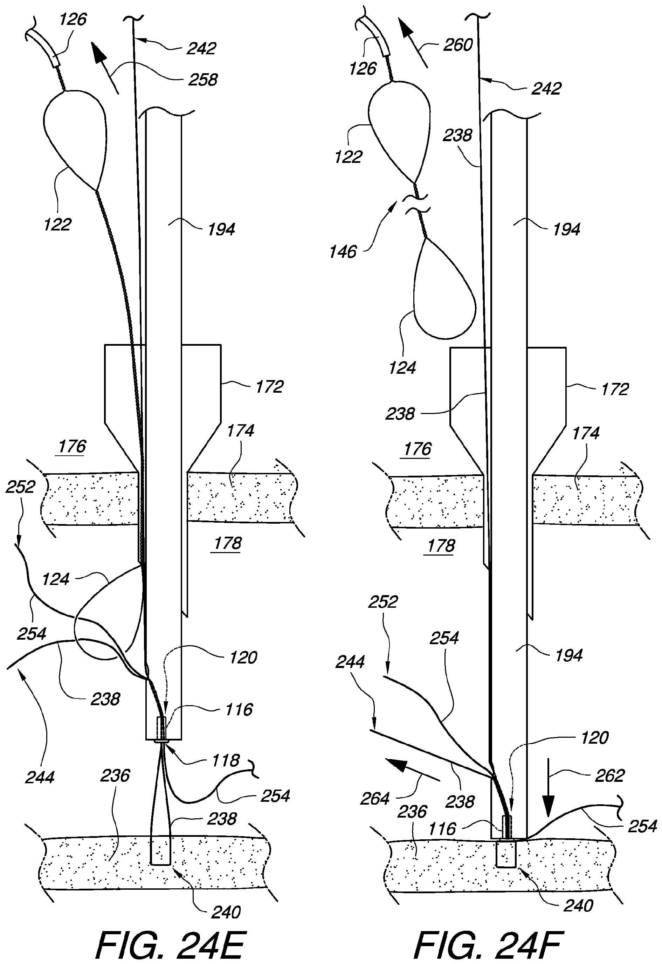

As illustrated in FIG. 24E, the handle 126 has been moved 258 away from the suture fastener 116 far enough to cause the distal suture engaging loop 124, and consequently the second end 244 of the first suture 238 and the first end 252 of the second suture 254 snared in that loop 124, to move through the suture fastener 116 from an entrance 118 to an exit 120 of the suture fastener 116. This second movement 258 of the handle 126 is enough to pull the distal suture engaging loop 124 through the suture fastener 116, but not enough to pull the distal loop 124 out of the patient. Accordingly, the second end 244 of the first suture 238 and the first end 252 of the second suture 254 remain available inside 178 the body.

After the second end 244 of the first suture 238 and the first end 252 of the second suture 254 have been pulled by the distal suture engaging loop 124 through the suture fastener 116, the second end 244 of the first suture 238 and the first end 252 of the second suture 254 may be removed from the loop 124 internally by the surgeon using MIS surgical tools at his or her disposal, whether manually or robotically operated. As illustrated in FIG. 24F, the surgical snare assembly 146 can be pulled 260 out of the cannula 172, while the first end 242 of the first suture 238 may be anchored or held outside 176 the body by a surgical assistant. The suture fastener applicator 194 can be moved 262 towards the stitch 240, and the surgeon can internally tension 264 the second end 244 of the first suture 238 to remove slack in the suture 238 and position the suture fastener 116 into a place where the suture stitch 240 can be held properly. It is very important that the tension on the first suture 238 is not too light, or the stitch 240 may have too much play. Similarly, it is important that the tension on the first suture 238 is not too strong in order to avoid tissue ischemia. The surgeon may also hold the first end 252 of the second suture 254 off to the side (but not necessarily under tension). Using the surgical suture snare and methods described herein, the surgeon can control the tension on the stitched suture 238 internally, even when operating through an MIS robotic interface, while a surgical assistant merely anchors or holds the other end of the suture outside the patient. Such methods, and their equivalents obviate the need for a second surgeon to assist with suture tensioning.

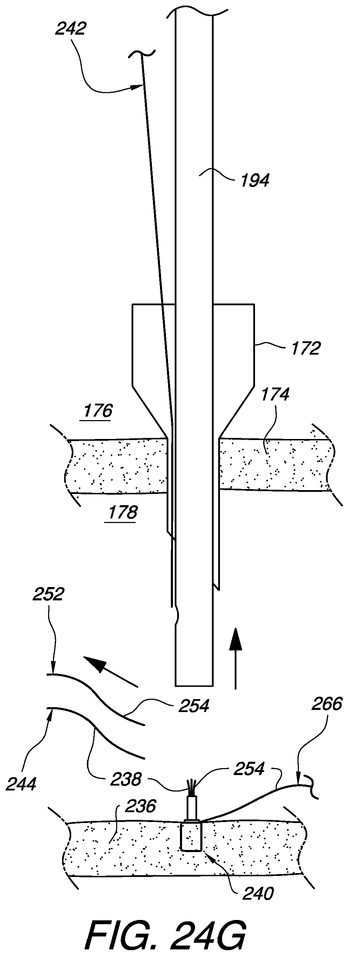

When the surgeon determines that the tension in at least the first suture 238 is correct, the surgeon or the surgical assistant can activate the suture fastener applicator 194 to attach the suture fastener 116 to the one or more sutures 238, 254 therein. The suture fastener applicator 194 may also be configured to cut the sutures on the exit 120 side of the suture fastener 116. Optionally, the sutures 238, 254 may be similarly cut with a separate MIS tool. The result is illustrated in FIG. 24G, where the suture fastener 116 is in place, successfully attached 1) to suture 238 to hold stitch 240 in place, and 2) to suture 254 to provide an anchored end for the free end 266 of the second suture 254. The former first end 242 of the first suture 238, now cut loose, can be removed by someone outside 176 the body. The second end 244 of the first suture 238 and the first end 252 of the second suture 254, also cut loose, can be removed by the surgeon using an MIS tool, and the suture fastener applicator 194 may be removed from the patient's body.

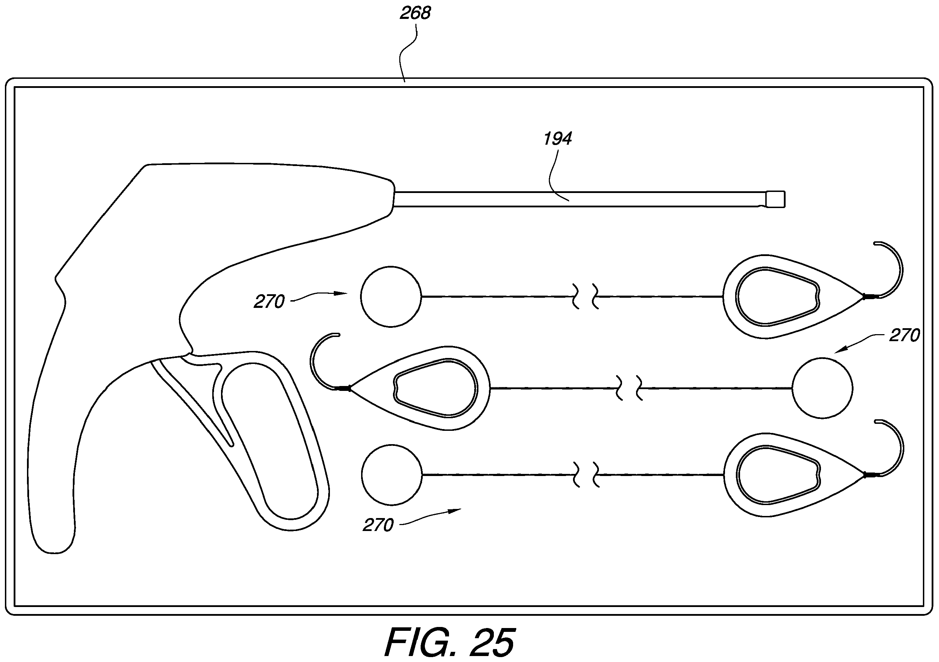

Since one or more surgical snare assemblies, such as those discussed above and their equivalents, are likely to be used with a suture fastener applicator, the two items may advantageously be manufactured and sold together as a kit. FIG. 25 illustrates one example of a surgical kit 268 having a suture fastener applicator 194 and at least one surgical snare assembly 270. Each of the one or more surgical snare assemblies 270 can be configured according to the embodiments of surgical snare assemblies discussed previously, as well as their equivalents.

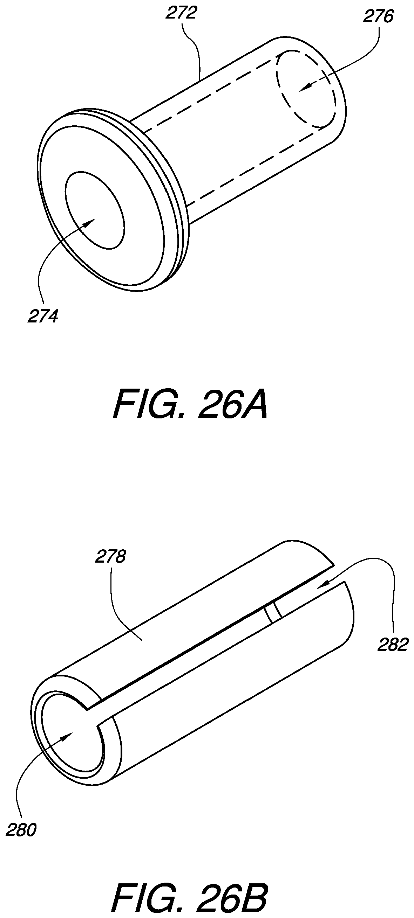

Each of the surgical snare assemblies discussed herein include a suture fastener having an entrance and an exit. For example, the suture fastener 272 illustrated in FIG. 26A has an entrance 274 and an exit 276. In the embodiment of FIG. 26A, the entrance 274 and the exit 276 are separate openings on the exterior surface of the suture fastener 272. In other embodiments, however, such as for the suture fastener 278 illustrated in FIG. 26B, the entrance 280 and the exit 282 are defined by the suture fastener as one continuous exterior opening on the suture fastener 278. Many other types of suture fasteners, of varying sizes, configurations, and features are known to those skilled in the art and it will be readily apparent to those skilled in the art that the embodiments herein may incorporate a wide variety of additional suture fastener types and designs.

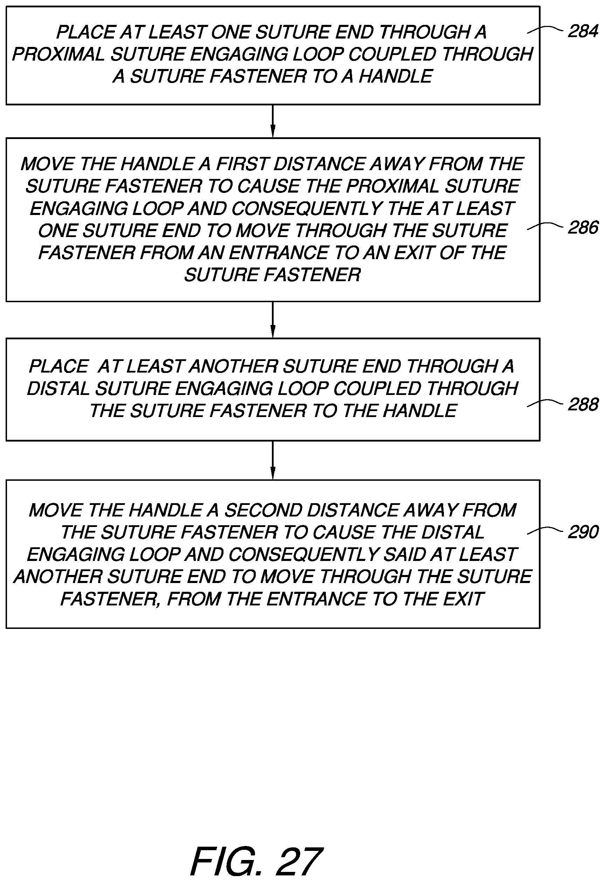

FIGS. 27-30 illustrate examples of suturing methods based on the examples discussed above. In the example of FIG. 27, in step 284, at least one suture end is placed through a proximal suture engaging loop coupled through a suture fastener to a handle. In step 286, the handle is moved a first distance away from the suture fastener to cause the proximal suture engaging loop and consequently the at least one suture end to move through the suture fastener from an entrance to an exit of the suture fastener. In step 288, the at least another suture end is placed through a distal suture engaging loop coupled through the suture fastener to the handle. Finally, in step 290, the handle is moved a second distance away from the suture fastener to cause the distal engaging loop and consequently said at least another suture end to move through the suture fastener, from the entrance to the exit.

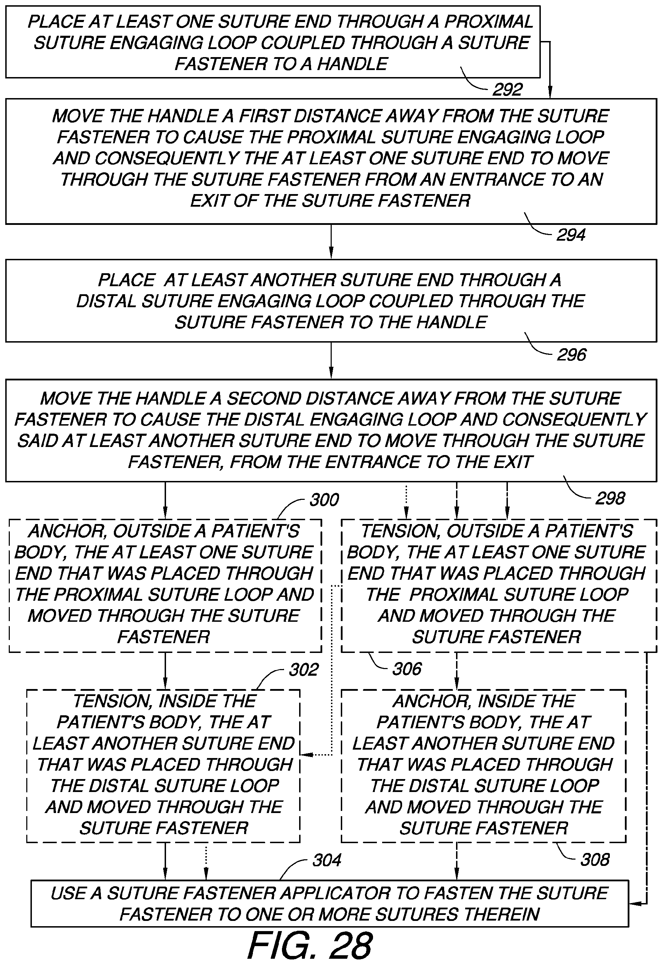

FIG. 28 illustrates another suturing method example. In step 292, at least one suture end is placed through a proximal suture engaging loop coupled through a suture fastener to a handle. In step 294, the handle is moved a first distance away from the suture fastener to cause the proximal suture engaging loop and consequently the at least one suture end to move through the suture fastener from an entrance to an exit of the suture fastener. In step 296, the at least another suture end is placed through a distal suture engaging loop coupled through the suture fastener to the handle. In step 298, the handle is moved a second distance away from the suture fastener to cause the distal engaging loop and consequently said at least another suture end to move through the suture fastener, from the entrance to the exit. At this point, there are four optional paths which can be taken before the final step 304. As part of a first possible path, in step 300, the at least one suture end that was placed through the proximal suture loop and moved through the suture fastener is anchored or otherwise held outside a patient's body. In step 302, the at least another suture end that was placed through the distal suture loop and moved through the suture fastener is tensioned inside the patient's body. Finally, in step 304, a suture fastener applicator is used to fasten the suture fastener to one or more sutures therein.

As part of a second possible path following step 298, in step 306 the at least one suture end that was placed through the proximal suture loop and moved through the suture fastener is tensioned outside the patient's body. Then, in step 302, the at least another suture end that was placed through the distal suture loop and moved through the suture fastener is tensioned inside the patient's body. Finally, in step 304, a suture fastener applicator is used to fasten the suture fastener to one or more sutures therein.

As part of a third possible path following step 298, in step 306 the at least one suture end that was placed through the proximal suture loop and moved through the suture fastener is tensioned outside the patient's body. In step 308, the at least another suture end that was placed through the distal suture loop and moved through the suture fastener is anchored inside the patient's body. Finally, in step 304, a suture fastener applicator is used to fasten the suture fastener to one or more sutures therein.

As part of a fourth possible path following step 298, in step 306 the at least one suture end that was placed through the proximal suture loop and moved through the suture fastener is tensioned outside the patient's body. Finally, in step 304, a suture fastener applicator is used to fasten the suture fastener to one or more sutures therein.

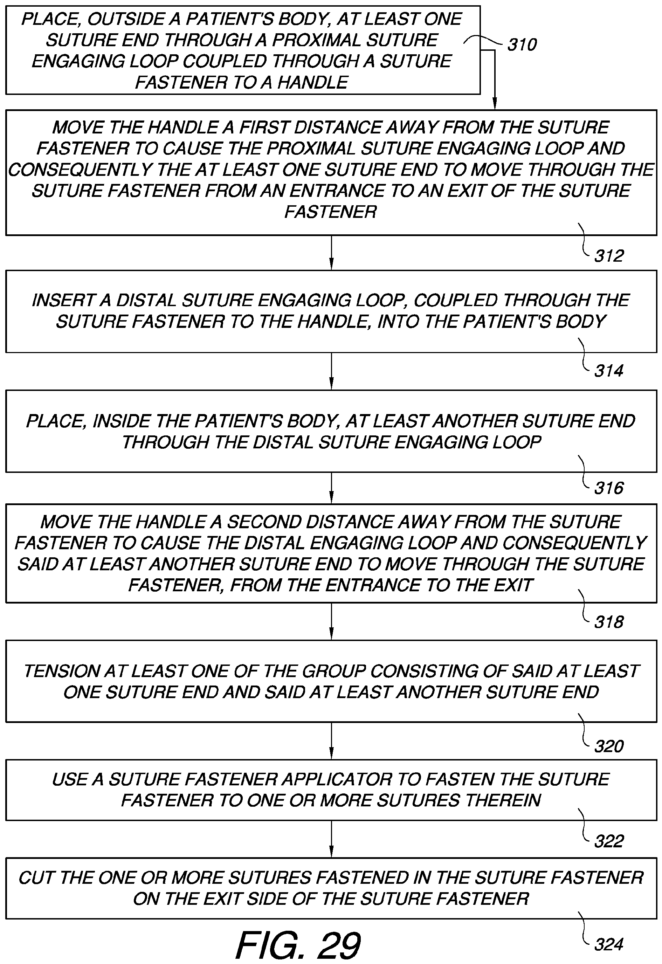

FIG. 29 illustrates another suturing method example. In step 310 at least one suture end is placed, outside a patient's body, through a proximal suture engaging loop coupled through a suture fastener to a handle. In step 312, the handle is moved a first distance away from the suture fastener to cause the proximal suture engaging loop and consequently the at least one suture end to move through the suture fastener from an entrance to an exit of the suture fastener. In step 314, a distal suture engaging loop, coupled through the suture fastener to the handle, is inserted into the patient's body. In step 316, at least another suture end is placed, inside the patient's body, through the distal suture engaging loop. In step 318, the handle is moved a second distance away from the suture fastener to cause the distal engaging loop and consequently said at least another suture end to move through the suture fastener, from the entrance to the exit. In step 320, at least one of the group consisting of said at least one suture end and said at least another suture end is tensioned. In step 322, a suture fastener applicator is used to fasten the suture fastener to one or more sutures therein. Finally, in step 324, the one or more sutures fastened in the suture fastener are cut on the exit side of the suture fastener.

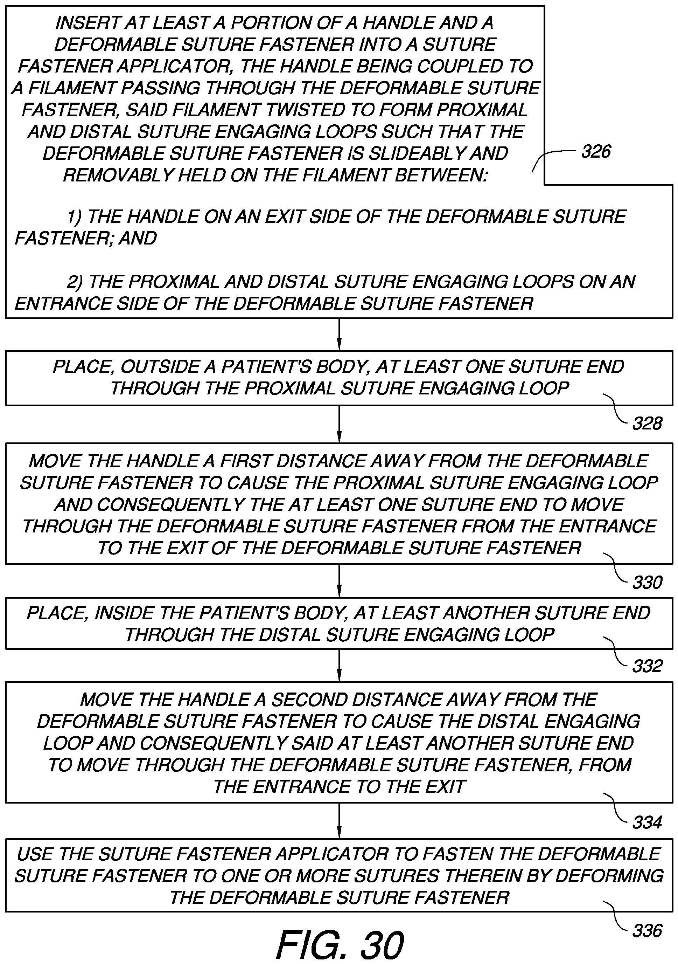

FIG. 30 illustrates another suturing method example. In step 326 at least a portion of a handle and a deformable suture fastener are inserted into a suture fastener applicator, the handle being coupled to a filament passing through the deformable suture fastener, said filament twisted to form proximal and distal suture engaging loops such that the deformable suture fastener is slideably and removably held on the filament between 1) the handle on an exit side of the deformable suture fastener and 2) the proximal and distal suture engaging loops on an entrance side of the deformable suture fastener. In step 328, at least one suture end is placed, outside a patient's body, through the proximal suture engaging loop. In step 330, the handle is moved a first distance away from the deformable suture fastener to cause the proximal suture engaging loop and consequently the at least one suture end to move through the deformable suture fastener from the entrance to the exit of the deformable suture fastener. In step 332, at least another suture end is placed, inside the patient's body, through the distal suture engaging loop. In step 334, the handle is moved a second distance away from the deformable suture fastener to cause the distal engaging loop and consequently said at least another suture end to move through the deformable suture fastener, from the entrance to the exit. Finally, in step 336, the suture fastener applicator is used to fasten the deformable suture fastener to one or more sutures therein by deforming the deformable suture fastener.

Various advantages of a multiple loop surgical snare assembly and suturing methods thereof have been discussed above. Embodiments discussed herein have been described by way of example in this specification. It will be apparent to those skilled in the art that the forgoing detailed disclosure is intended to be presented by way of example only, and is not limiting. Various alterations, improvements, and modifications of all the disclosed embodiments and their equivalents will occur and are intended to those skilled in the art, though not expressly stated herein. As just one example, although the embodiments illustrated herein include two suture engaging loops (one proximal and one distal), other embodiments may include any number of two or more suture engaging loops. Such a surgical snare assembly might still have a handle, a suture fastener having an entrance and an exit, and two or more suture engaging loops coupled to the handle. In these types of embodiments, each of the two or more suture engaging loops would extend different distances past the handle. The handle would be configured such that movement of the handle away from the suture fastener causes each of the two or more suture engaging loops to move through the suture fastener from the entrance to the exit at different distances of handle movement. These and any other alterations, improvements, and modifications are intended to be suggested hereby. Additionally, the recited order of processing elements or sequences, or the use of numbers, letters, or other designations therefore, is not intended to limit the claims to any order, except as may be specified in the claims. Accordingly, the invention is limited only by the following claims.

## Claims

1. A surgical snare assembly (144, 146, 150, 156), comprising:
a suture fastener (116) having an entrance (118) and an exit (120);
a proximal suture engaging loop (122);
a distal suture engaging loop (124); and
a handle (126) which is directly coupled to the proximal suture engaging loop (122) and coupled to the distal suture engaging loop (124), and configured such that:
movement of the handle (126) a first distance away from the suture fastener (116) causes the proximal suture engaging loop (122) to move through the suture fastener (116) from the entrance (118) to the exit (120); and
movement of the handle (126) a second distance away from the suture fastener (116) causes the distal suture engaging loop (124) to move through the suture fastener (116) from the entrance (118) to the exit (120);
**characterized in that** the distal suture engaging loop (124) is indirectly coupled to the handle (126)
by the proximal suture engaging loop (122) and a twisted filament (148) extending between the proximal suture engaging loop (122) and the distal suture engaging loop (124), or
by the proximal suture engaging loop (122) and at least one or more filaments (154) extending between the proximal suture engaging loop (122) and the distal suture engaging loop (124).

2. The surgical snare assembly of claim 1, wherein the handle (126) is configured to guide the suture fastener (116) into a suture fastener applicator (194).

3. The surgical snare assembly of claim 2, wherein the handle (126) is configured to guide the suture fastener (116) into the suture fastener applicator (194) before movement of the handle (126) the first or the second distance away from the suture fastener (116).

4. The surgical snare assembly of claim 1, wherein the entrance (118) of the suture fastener (116) and the exit (120) of the suture fastener (116) are defined by the suture fastener (116) as one continuous exterior opening on the suture fastener (116).

5. The surgical snare assembly of claim 1, wherein the proximal suture engaging loop (122) is differentiable from the distal suture engaging loop (124) by at least one characteristic selected from the group consisting of a color, a texture, a material type, and a material dimension.

6. The surgical snare assembly of claim 1, wherein the suture fastener (116) comprises a deformable metal fastener.

7. The surgical snare assembly of claim 1, further comprising a proximal holder on which at least a portion of the proximal suture engaging loop (122) is removably supported.

8. The surgical snare assembly of claim 1, further comprising a distal holder on which at least a portion of the distal suture engaging loop (124) is removably supported.

## Patentansprüche

1. Chirurgische Schlingenanordnung (144, 146, 150, 156), umfassend:
eine Nahtbefestigungsvorrichtung (116), die einen Eingang (118) und einen Ausgang (120) aufweist;
eine proximale Nahteingriffsschlaufe (122);
eine distale Nahteingriffsschlaufe (124); und
einen Griff (126), der direkt mit der proximalen Nahteingriffsschlaufe (122) gekoppelt ist und mit der distalen Nahteingriffsschlaufe (124) gekoppelt ist und der so ausgestaltet ist, dass:
eine Bewegung des Griffs (126) um eine erste Entfernung von der Nahtbefestigungsvorrichtung (116) weg bewirkt, dass sich die proximale Nahteingriffsschlaufe (122) durch die Nahtbefestigungsvorrichtung (116) von dem Eingang (118) zu dem Ausgang (120) bewegt; und
eine Bewegung des Griffs (126) um eine zweite Entfernung von der Nahtbefestigungsvorrichtung (116) weg bewirkt, dass sich die distale Nahteingriffsschlaufe (124) durch die Nahtbefestigungsvorrichtung (116) von dem Eingang (118) zu dem Ausgang (120) bewegt;
**dadurch gekennzeichnet, dass** die distale Nahteingriffsschlaufe (124) indirekt mit dem Griff (126) gekoppelt ist
durch die proximale Nahteingriffsschlaufe (122) und einen verdrillten Faden (148), der sich zwischen der proximalen Nahteingriffsschlaufe (122) und der distalen Nahteingriffsschlaufe (124) erstreckt, oder
durch die proximale Nahteingriffsschlaufe (122) und mindestens einen oder mehrere Fäden (154), die sich zwischen der proximalen Nahteingriffsschlaufe (122) und der distalen Nahteingriffsschlaufe (124) erstrecken.

2. Chirurgische Schlingenanordnung nach Anspruch 1, wobei der Griff (126) so ausgestaltet ist, dass er die Nahtbefestigungsvorrichtung (116) in einen Nahtbefestigungsapplikator (194) führt.

3. Chirurgische Schlingenanordnung nach Anspruch 2, wobei der Griff (126) so ausgestaltet ist, dass er die Nahtbefestigungsvorrichtung (116) in den Nahtbefestigungsapplikator (194) führt, bevor der Griff (126) um die erste oder die zweite Entfernung von der Nahtbefestigungsvorrichtung (116) weg bewegt wird.

4. Chirurgische Schlingenanordnung nach Anspruch 1, wobei der Eingang (118) der Nahtbefestigungsvorrichtung (116) und der Ausgang (120) der Nahtbefestigungsvorrichtung (116) durch die Nahtbefestigungsvorrichtung (116) als eine durchgehende äußere Öffnung an der Nahtbefestigungsvorrichtung (116) definiert sind.

5. Chirurgische Schlingenanordnung nach Anspruch 1, wobei die proximale Nahteingriffsschlaufe (122) von der distalen Nahteingriffsschlaufe (124) durch mindestens ein Merkmal unterscheidbar ist, das ausgewählt ist aus der Gruppe, die aus Farbe, Textur, Materialtyp und Materialabmessung besteht.

6. Chirurgische Schlingenanordnung nach Anspruch 1, wobei die Nahtbefestigungsvorrichtung (116) eine verformbare Metallbefestigungsvorrichtung umfasst.

7. Chirurgische Schlingenanordnung nach Anspruch 1, die des Weiteren eine proximale Haltevorrichtung umfasst, an der zumindest ein Teil der proximalen Nahteingriffsschlaufe (122) abnehmbar abgestützt ist.

8. Chirurgische Schlingenanordnung nach Anspruch 1, des Weiteren umfassend eine distale Haltevorrichtung, an der zumindest ein Teil der distalen Nahteingriffsschlaufe (124) abnehmbar abgestützt ist

## Revendications

1. Ensemble formant collet chirurgical (144, 146, 150, 156), comprenant :
un fixateur de suture (116) ayant une entrée (118) et une sortie (120) ;
une boucle d'engagement de suture proximale (122) ;
une boucle d'engagement de suture distale (124) ; et
une manette (126) qui est directement couplée à la boucle d'engagement de suture proximale (122) et couplée à la boucle d'engagement de suture distale (124), et configurée de telle façon que :
un mouvement de la manette (126) sur une première distance en éloignement du fixateur de suture (116) amène la boucle d'engagement de suture proximale (122) à se déplacer à travers le fixateur de suture (116) depuis l'entrée (118) jusqu'à la sortie (120) ; et
un mouvement de la manette (126) sur une seconde distance en éloignement du fixateur de suture (116) amène la boucle d'engagement de suture distale (124) à se déplacer à travers le fixateur de suture (116) depuis l'entrée (118) jusqu'à la sortie (120) ;
**caractérisé en ce que** la boucle d'engagement de suture distale (124) est indirectement couplée à la manette (126)
soit par la boucle d'engagement de suture proximale (122) et un filament torsadé (148) s'étendant entre la boucle d'engagement de suture proximale (122) et la boucle d'engagement de suture distale (124),
soit par la boucle d'engagement de suture proximale (122) et au moins un ou plusieurs filaments (154) s'étendant entre la boucle d'engagement de suture proximale (122) et la boucle d'engagement de suture distale (124).

2. Ensemble formant collet chirurgical selon la revendication 1, dans lequel la manette (126) est configurée pour guider le fixateur de suture (116) jusque dans un applicateur de fixateur de suture (194).

3. Ensemble formant collet chirurgical selon la revendication 2, dans lequel la manette (126) est configurée pour guider le fixateur de suture (116) jusque dans l'applicateur de fixateur de suture (194) avant un mouvement de la manette (126) sur la première ou sur la seconde distance en éloignement du fixateur de suture (116).

4. Ensemble formant collet chirurgical selon la revendication 1, dans lequel l'entrée (118) du fixateur de suture (116) et la sortie (120) du fixateur de suture (116) sont définies par le fixateur de suture (116) comme une ouverture extérieure continue sur le fixateur de suture (116).

5. Ensemble formant collet chirurgical selon la revendication 1, dans lequel la boucle d'engagement de suture proximale (122) est capable d'être différenciée vis-à-vis de la boucle d'engagement de suture distale (124) par au moins une caractéristique sélectionnée parmi le groupe comprenant : couleur, texture, type de matériau, et dimension matérielle.

6. Ensemble formant collet chirurgical selon la revendication 1, dans lequel le fixateur de suture (116) comprend un fixateur en métal déformable.

7. Ensemble formant collet chirurgical selon la revendication 1, comprenant en outre un dispositif de maintien proximal sur lequel au moins une portion de la boucle d'engagement de suture proximale (122) est supportée de façon amovible.

8. Ensemble formant collet chirurgical selon la revendication 1, comprenant en outre un dispositif de maintien distal sur lequel au moins une portion de la boucle d'engagement de suture distale (124) est supportée de façon amovible.
